# EUROPEAN PATENT SPECIFICATION

(11) **EP 4 404 892 B1**
(45) Date of publication and mention of the grant of the patent: **03.12.2025**
(21) Application number: 22871124.8
(22) Date of filing: 21.09.2022
(51) Int. Cl.: A61F 13/496, A41B 9/12, A61F 13/49

(54) **PROTECTIVE GARMENT**
SCHUTZKLEIDUNG
VÊTEMENT DE PROTECTION

(30) Priority: 21.09.2021 SE 2151152
(43) Date of publication of application: 31.07.2024
(60) Divisional application: 24222512.6
(73) Proprietor: AB Lindex, 401 23 Göteborg (SE)
(72) Inventor: DREVIK, Solgun, 514 93 Ambjörnarp (SE); GREIJER, Unn, 414 58 Göteborg (SE); JOHANSSON, Jenny, 438 32 Landvetter (SE); LO, Ebba, 134 32 Gustavsberg (SE)
(74) Representative: Noréns Patentbyrå AB
(86) International application number: PCT/SE2022/050828
(87) International publication number: WO 2023/048620

(56) References cited:
- WO-A1-2004/075801
- WO-A1-99/25298
- CN-A- 1 098 897
- CN-A- 1 098 897
- CN-U- 214 127 031
- JP-A- 2002 095 688
- JP-A- 2003 171 802
- KR-B1- 101 880 530
- KR-B1- 101 956 752
- US-A1- 2006 142 725
- US-B1- 6 652 503

## Description

The present invention relates to a protective garment, such as a pantie, a pair of trousers or other legwear with or without an upper/torso part. The protective garment may also be a skirt, a dress or similar, having a protective crotch part. Specifically, the protective garment is designed to protect against leakage of bodily fluids such as menses, urine and similar when worn by a user.

Specifically, the present protective garment may be designed to be suitable for female users.

There are many known solutions to the problem of legwear absorption and leakage protection in various situations.

For instance, GB 2282522 A discloses a sanitary pantie featuring an absorbent body and various elastic elements extending past a crotch part of the pantie to achieve sealing against the skin of the user wearing the pantie, making sure that all bodily fluids are absorbed by said absorbent body.

As another example, WO 2013/148749 discloses a protective pantie provided with an elastic element pulling an integrated sanitary pad upwards against the skin of a user wearing the pantie, so as to achieve adequate contact between the user and the sanitary pad.

Additional examples of known solutions include WO 2012/047650 A1 and US 10888470 B2 describing a washable panty with absorbing material; and WO 2013/147658 A1 and GB 2435403 A, describing panties with separate pads.

These solutions may perform acceptably in terms of leakage protection and absorption.

However, there is a need for a protective garment of said general type offering an improved user experience when worn, such as in terms of aesthetical appearance and/or comfort, with maintained absorption and protection against leakage. In particular, there is a need for a washable garment with these properties, that can be used repeatedly to provide leakage protection, and be washed in between uses. KR10195675281 discloses a protective garment according to the preamble of claim 1.

The present invention solves the above described problems.

Hence, the invention relates to a protective garment in the form of a pantie, a pair of trousers or other legwear with or without an upper/torso part, the protective garment being a washable garment arranged for repeated use comprising a front part; a back part; a waist; and a crotch part having a leak-proof body, the protective garment being characterised in that
the protective garment comprises a dorsal shaping element, arranged along the intergluteal cleft of a standing user wearing the protective garment, fastened to or constituting an integrated part of the leak-proof body and fastened to or constituting an integrated part of the back part,
   , in that
the back part comprises a panel part separating the dorsal shaping element from the waist so that the dorsal shaping element does not extend all the way up the waist of the protective garment worn by the standing user, in that

the dorsal shaping element is arranged so that it provides locally differing properties of the material o the protective garment so that, when a dorsal/upward force is applied to the dorsal shaping element via the panel part as a result of the waist being pulled upwards in relation to said user wearing the protective garment, it in turn forces a part of the crotch part extending along the intergluteal cleft of said user upwards, and in that
said locally differing properties are provided by the dorsal shaping element comprising one or several of applied silicone or a different glue; a weld; a stitching; a fibre flocking; a velvet material; injected molten plastic material, locally varied seamless knitting; and a dorsally elongation of the crotch part overlapping with and fastened to the back part, and in that said dorsal shaping element constitutes, is comprised in or comprises a joining area, joining together the leak-proof body and the back part.

In some embodiments, the dorsal shaping element extends dorsally and upwards along the intergluteal cleft of the standing user wearing the protective garment.

In some embodiments, the dorsal shaping element has a length of at least 3 cm running along the intergluteal cleft of the standing user wearing the protective garment.

In some embodiments, the protective garment further comprises a pair of elongated ventral shaping elements, arranged to extend on either lateral side of the labia of said standing user wearing the protective garment, ventrally from the dorsal shaping element, and arranged so that, when a ventral/upward force is applied to the ventral shaping elements via the front part as a result of the waist being pulled upwards in relation to said user wearing the protective garment, it is in turn forced upwards.

In some embodiments, said ventral shaping elements connect to either side of a ventral end edge of the crotch part and/or a ventral end edge of the leak-proof body and/or a ventral edge of a liquid-absorbing layer comprised in the leak-proof body.

In some embodiments, said dorsal shaping element is directly connected to said ventral shaping elements.

In some embodiments, said dorsal shaping element, together with said ventral shaping elements, forms a "Y"-shape.

In some embodiments, said dorsal shaping element comprises an integrated part of a knitted or weaved textile layer of the protective garment, produced by, during manufacturing, locally varying the properties of said textile layer by changing the weaving or knitting procedure.

In some embodiments, said dorsal shaping element is elastic and arranged to stretch as a result of said pulling upwards of the waist.

In some embodiments, said dorsal shaping element is inelastic.

In some embodiments, said joining area joins together a liquid-absorbing layer comprised in the leak-proof body and the back part.

In some embodiments, said dorsal shaping element comprises joining means, such as a stitched joint, glue joint or weld joint, the joining means joining the leak-proof body to the back part.

In some embodiments, said joining means is arranged at a dorsal end edge of a liquid-absorbing layer of the leak-proof body.

In some embodiments, said dorsal shaping element comprises a part being separate from the crotch part and the back part, said separate part connecting the crotch part to the back part.

In some embodiments, said crotch part, such as a liquid-absorbing layer comprised in the crotch part, comprises a ventral end edge, generally laterally elongated and having a convex shape softly bulging ventrally/upwards in relation to said standing user wearing the protective garment.

In some embodiments, said bulge of said convex shape extends at least 3 cm ventrally/upwards in relation to said standing user wearing the protective garment.

In some embodiments, said crotch part, such as a liquid-absorbing layer comprised in the crotch part, comprises a dorsal end edge, generally laterally elongated and comprising a direction change, at the intergluteal cleft of said standing user wearing the protective garment, the direction change defining an acute angle pointing upwards/dorsally in relation to the user.

In some embodiments, said dorsal shaping element comprises a straight part, extending at least 1 cm, such as between 1 cm and 30 cm, dorsally from the apex of said acute angle.

In some embodiments, the leak-proof body and/or a central part of the leak-proof body being defined between a pair of dorsally-ventrally extending fold lines in the leak-proof body has a laterally local narrowest part arranged between the legs, such as at the gracilis, of the standing user wearing the protective garment.

In some embodiments, side edges of the leak-proof body and/or of said central part of the leak-proof body diverge laterally outwards from said laterally narrowest part, in both a dorsal direction and in a ventral direction in relation to the standing user wearing the protective garment.

In some embodiments, said side edges of the leak-proof body both extend between a dorsal end edge of a liquid-absorbing layer comprised in the leak-proof body and a ventral end edge of said liquid-absorbing layer.

An unclaimed embodiment relates to a protective garment in the form of a pantie, a pair of trousers or other legwear with or without an upper/torso part, the protective garment comprising a front part; a back part; a waist; and a crotch part having a leak-proof body, the protective garment optionally being of the type described above, wherein the crotch part extends along a path along the sagittal plane of a standing user wearing the protective garment, at least part of said path extending along the intergluteal cleft of said standing user, and wherein the crotch part comprises, in order from an inner part of the crotch part to an outer part of the crotch part: a flexible first layer; a flexible second liquid-absorbing material layer, extending along said path; and a flexible third liquid barrier layer.

In some embodiments, the third layer extends along said path along the entire length of the extension along said path of said second layer and also along at least a part along said path not occupied by the second layer dorsally in said sagittal plane.

In some embodiments, the crotch part comprises a dorsal joining area joining together the first layer and the second layer, the dorsal joining area extending laterally in relation to said standing user wearing the protective garment and fastening the second layer to the first layer.

In some embodiments, the dorsal joining area fastens the second layer to the first layer along a dorsal end edge of the second layer.

In some embodiments, the dorsal joining area constitutes a liquid barrier.

In some embodiments, the dorsal joining area comprises a generally laterally elongated shape comprising a direction change, at the intergluteal cleft of said user, said direction change defining an acute angle pointing upwards/dorsally in relation to the user.

In some embodiments, the dorsal joining area comprises several generally laterally elongated and possibly separated shapes, distributed along the intergluteal cleft of said standing user wearing the protective garment, at least two of said several elongated shapes at least partly overlapping with the second layer.

In some embodiments, the crotch part comprises several liquid-absorbing layers, different ones of said shapes of the dorsal joining area joining together different sets of said liquid-absorbing layers to the first layer.

In some embodiments, a more dorsally arranged, in relation to the standing user wearing the protective garment, one of said shapes of the dorsal joining area joins together a smaller number of said liquid-absorbing layers than a more ventrally arranged one of said shapes of the dorsal joining area.

In some embodiments, the dorsal joining area comprises or constitutes the dorsal shaping element.

In some embodiments, the dorsal joining area comprises stitches joining together said first and second layers, the stitches not passing through the third layer.

In some embodiments, the second and third layers are fastened together along respective lateral side edges of the second and third layers.

In some embodiments, the third layer extends at least 25 cm along said path for a medium sized garment according to standard EN 13402.

In some embodiments, the crotch part comprises a ventral joining area joining together the first layer and the second layer, the ventral joining area extending laterally in relation to said standing user wearing the protective garment and fastening the second layer to the first layer.

In some embodiments, the ventral joining area fastens the second layer to the first layer along a ventral end edge of the second layer.

In some embodiments, the ventral joining area constitutes a liquid barrier.

In some embodiments, the ventral joining area comprises a generally laterally elongated shape having a convex shape softly bulging ventrally/upwards in relation to said standing user wearing the protective garment.

In some embodiments, the ventral joining area comprises several generally laterally elongated shapes, distributed along a line between the perineum and the bellybutton of said standing user wearing the protective garment, at least two of said several elongated shapes at least partly overlapping with the second layer.

In some embodiments, the crotch part comprises several liquid-absorbing layers, different ones of said shapes of the ventral joining area joining together different sets of said liquid-absorbing layers to the first layer.

In some embodiments, a more ventrally arranged, in relation to the standing user wearing the protective garment, one of said shapes of the ventral joining area joins together a smaller number of said liquid-absorbing layers than a more dorsally arranged one of said shapes of the ventral joining area.

In some embodiments, the second layer is arranged to move freely in relation to the third layer in a centrally located area of the crotch part, between the dorsal joining area and the ventral joining area.

In some embodiments, the second layer comprises a super-absorbing fibre material.

An unclaimed embodiment relates to a protective garment in the form of a pantie, a pair of trousers or other legwear with or without an upper/torso part, the protective garment comprising a front part; a back part; a waist; and a crotch part having a leak-proof body, the protective garment optionally being of the type described above, wherein the crotch part comprises, in order from an inner part of the crotch part to an outer part of the crotch part, a first distribution layer having relatively large pores; a second distribution layer having relatively small pores; a liquid-absorbing layer; and a liquid barrier.

An unclaimed embodiment relates to a protective garment in the form of a pantie, a pair of trousers or other legwear with or without an upper/torso part, the protective garment comprising a front part; a back part; a waist; and a crotch part having a leak-proof body, the protective garment optionally being of the type described above, wherein the crotch part comprises, in order from an inner part of the crotch part to an outer part of the crotch part, a first distribution layer being arranged with higher liquid transportation capacity in a direction perpendicular to the skin of the standing user wearing the protective garment as compared to in a direction parallel to said skin; a second distribution layer being arranged with a higher liquid transportation capacity in a direction parallel to said skin as compared to in a direction perpendicular to said skin; a liquid-absorbing layer; and a liquid barrier.

In some embodiments, the liquid-absorbing layer comprises a non-woven super-absorbent fibre material, the fibres of the fibre material being permanently bound to a flexible substrate material.

In some embodiments, the first and/or second distribution layer comprises a spacer material, arranged to distribute a liquid by capillary forces.

In some embodiments, the crotch part comprises a plurality of connections, such as stitches and/or welds, joining together the first distribution layer to at least one additional layer of the crotch part, the plurality of connections defining a connection pattern distributed across the first distribution layer distributed to cover at least 50% of the first distribution layer at a location of the genitalia of the standing user wearing the protective garment.

In some embodiments, said connections constitute a contact-limiting means for limiting a share of said first distribution layer being in direct contact with said user wearing the protective garment, by the first distribution layer locally bulging inwards towards the liquid barrier at the connection in question.

In some embodiments, the connection pattern comprises several different connections joining together the first distribution layer with different numbers of other layers selected from the set of the second distribution layer; any additional distribution layer comprised in the crotch part; the liquid-absorbing layer; and any additional liquid-absorbing layers comprised in the crotch part.

In some embodiments, the connection pattern comprises hydrophilic and/or hydrophobic connections, such as quilted joints comprising stitches using hydrophilic and/or hydrophobic thread.

In some embodiments, the connections do not penetrate through the liquid barrier.

An unclaimed embodiment relates to a protective garment in the form of a pantie, a pair of trousers or other legwear with or without an upper/torso part, the protective garment comprising a front part; a back part; a waist; and a crotch part having a leak-proof body, the protective garment optionally being of the type described above, wherein the crotch part comprises an inner surface layer, comprising flocked fibres. The flocked fibres are permanently fastened by flocking the fibres to a surface of a particular layer of the crotch part.

In some embodiments, the flocked fibres are permanently fastened to the inner layer of the crotch part, the inner surface layer being permeable to liquids.

In some embodiments, the flocked fibres are permanently fastened to a distribution layer of the crotch part, the inner surface layer being permeable to liquids.

In some embodiments, the flocked fibres are instead permanently fastened to a liquid-absorbing layer of the crotch part, the inner surface layer being permeable to liquids.

In some embodiments, the flocked fibres are instead permanently fastened to a liquid barrier layer of the crotch part.

In some embodiments, the inner surface layer comprises a flock pattern across the inner surface layer, said flock pattern comprising flocked and non-flocked areas.

In some embodiments, the flocked areas are distributed to constitute at least 20%, such as at least 30%, such as at least 50%, of the surface of the layer to which the flocked fibres are permanently fastened at a location of the genitalia of the standing user wearing the protective garment.

In some embodiments, said flocked fibres are or comprise hydrophilic fibres.

In some embodiments, the non-flocked areas are distributed to constitute at least 10%, such as at least 20%, such as at least 50%, of the surface of the layer to which the flocked fibres are permanently fastened at a location of the genitalia of the standing user wearing the protective garment.

In some embodiments, said flocked fibres comprise hydrophobic fibres.

In some embodiments, said flocked fibres comprises external fibres, arranged to be in direct contact with the skin of the standing user wearing the protective garment.

In some embodiments, said flocked fibres comprise both hydrophilic fibres and hydrophobic fibres, the hydrophilic and hydrophobic fibres both being flocked to the surface in question in a respective regular or random pattern covering a centrally located area of the crotch part covering the genitals of the standing user wearing the protective garment.

In some embodiments, said flocked fibres constitute a contact-limiting means for limiting a share of a layer of the crotch part being in direct contact with said user wearing the protective garment.

In some embodiments, said flocked fibres comprise external hydrophobic fibres arranged on an inside, facing the skin of said standing user wearing the protective garment, of said layer of the crotch part in turn being in direct contact with said user.

An unclaimed embodiment relates to a protective garment in the form of a pantie, a pair of trousers or other legwear with or without an upper/torso part, the protective garment comprising a front part; a back part; a waist; and a crotch part having a leak-proof body, the protective garment optionally being of the type described above, wherein the leak-proof body comprises a respective side part arranged on either lateral side of the genitals of a standing user wearing the protective garment, each side part being connected to the rest of the leak-proof body via a respective curved fold line a centre of curvature of which fold line lies laterally outside of the leak-proof body, and wherein the respective end points of each of said fold lines are interconnected by a flexible element arranged with an elastic property and/or a length resulting in that the flexible element in question applies a force pulling said end points together when the protective garment is worn by the standing user, and as a result of said force pushing the side part in question upwards.

In some embodiments each of said side parts is only connected to the rest of the protective garment along said respective curved fold line.

In some embodiments said pulling force forces said flexible elements upwards in relation to the standing user wearing the protective garment, forming a cup-shape covering the genitalia of said standing user.

In some embodiments a minimum lateral distance between said two fold lines is at the most 5 cm, such as at the most 4 cm.

In some embodiments a minimum lateral distance between said two fold lines is at least 2 cm, such as at least 2.5 cm.

In some embodiments each of said side parts comprises a liquid barrier material.

In some embodiments each of said fold lines is constituted by a stitched seam, a weld line or any other means locally compressing the material of the leak-proof body along the fold line in question, and/or a cut-out arranged perpendicularly to the fabric to define the fold line as an elongated depression, and/or a joint between two separate but fastened together pieces of fabric to define the fold line as an elongated depression.

In some embodiments the crotch part is a separate part from said front part and/or said back part, the different parts of the protective garment being permanently joined together, such as using stitching, welding or gluing.

In some embodiments at least one layer of the crotch part is manufactured from the same integrated flexible material as at least one layer of said front part and/or at least one layer of said back part, a peripheral shape of the said at least one layer of the crotch part being defined, in relation to said at least one layer of said front part and/or of said back part, by areas of varying stitching types, stitching, flocking, gluing, bonding and/or welding.

In the following, the invention will be described in detail, with reference to exemplifying embodiments of the invention and to the enclosed drawings, wherein:
Figures 1a-1d are flat views of respective protective garments;
Figure 1e is a perspective view of the protective garment illustrated in Figure 1d;
Figure 2 is a horizontal plane cross-section illustrating a dorsal shaping element of a protective garment;
Figure 3a is a flat view of a protective garment;
Figure 3b is a simplified cross-sectional view, showing a vertical cross-section, seen in a sagittal plane of a user wearing the protective garment, of the protective garment shown in Figure 3a;
Figure 3c is a perspective view of the protective garment illustrated in Figures 3a and 3b;
Figure 4a is a simplified cross-sectional view, showing a vertical cross-section, seen in a sagittal plane of a user wearing the protective garment, of a protective garment;
Figure 4b is a simplified cross-sectional view, showing a vertical cross-section, seen in a sagittal plane of a user wearing the protective garment, of another protective garment;
Figure 4c is a plan view of a crotch part of the garment illustrated in Figure 4b;
Figures 4d and 4e correspond to Figure 4a, but showing other examples of protective garments;
Figures 5a and 5b correspond to Figure 4a, but showing other examples of protective garments;
Figure 6a is a flat view of a protective garment;
Figure 6b is a simplified cross-sectional view, showing a vertical cross-section of the protective garment as seen in a frontal plane of a user wearing the protective garment;
Figure 6c is a perspective view of the protective garment illustrated in Figure 6a;
Figures 7a-7b show front and back sides, respectively, of a protective garment; and
Figures 7c-7d show front and back sides, respectively, of a protective garment.

It is noted that the Figures illustrate different exemplifying embodiments of the present invention. These embodiments have been selected in order to clearly illustrate various aspects and features described herein, and it is understood that individual features and properties illustrated in different Figures can be combined in any way, subject to compatibility.

It is further noted that the Figures share the same reference numerals for same and corresponding parts. The Figures are intended to illustrate the principles used by the various embodiments of the present invention, and are not necessarily to scale.

It is further noted that the present solutions circle around a number of different cooperating features that can be combined in various ways so as to solve the initially described problem. This will become clear from the following description of a number of embodiment examples of the present invention.

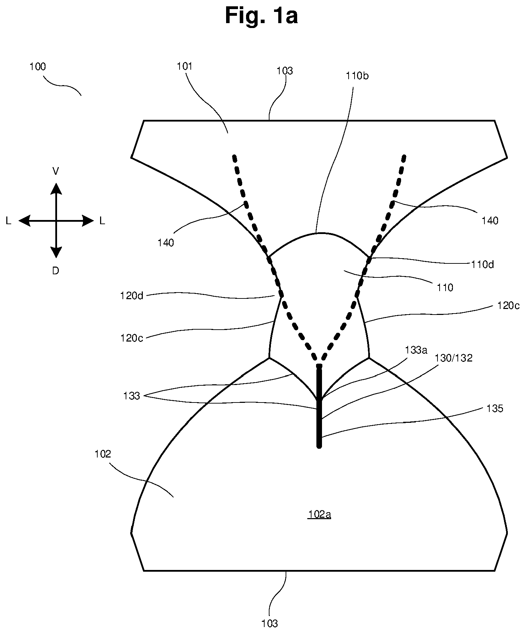

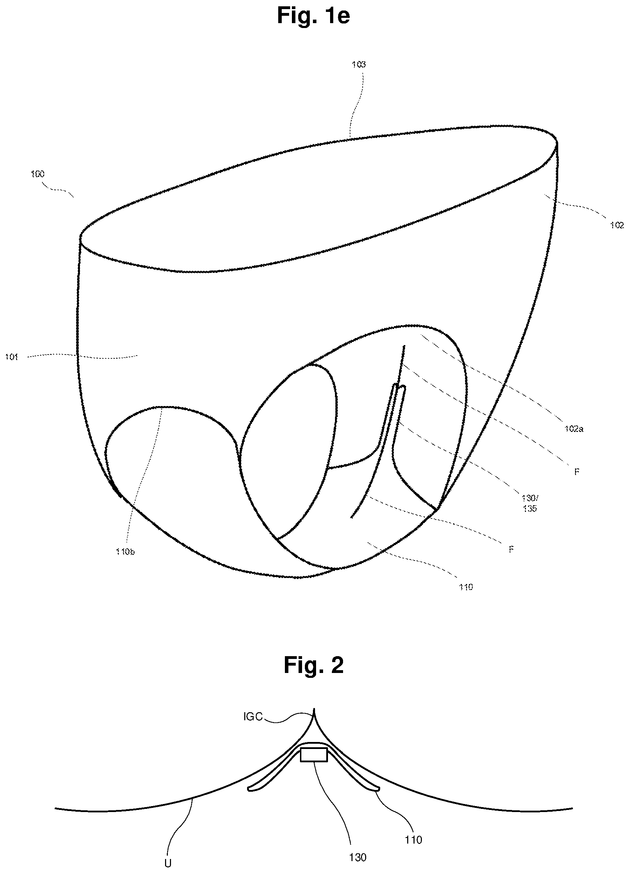

Hence, Figure 1e shows a protective garment 100 according to the invention in a perspective view, while Figures 1a-1d show the same protective garment 100 in respective flattened views.

In Figures 1a, lateral directions L are shown, as well as a dorsal direction D and a ventral direction V. These directions apply correspondingly the other Figures, as applicable. Furthermore, UP denotes the upwards direction, in relation to the standing, upright user wearing the protective garment 100.

The protective garment 100 is in the form of a pantie, a pair of trousers or other legwear, such as tights, shorts or a skirt with integrated panty. Furthermore, the protective garment 100 can be designed with or without an upper/torso part. For instance, the protective garment 100 can be a body with integrated panty, or a jumpsuit. Moreover, the protective garment 100 can be a skirt, a dress, a piece of active wear, a piece of shapewear, or a swimwear. Of course, it is also possible that the protective garment 100 is of more than one of these exemplifying types at once, such as a swimwear in the form of a skirt.

The protective garment 100 comprises a front part 101, a back part 102, a waist 103 and a crotch part 110. It is understood that these parts 101, 102, 103, 110 may each be separate and joined together parts of the garment 100, or that two or more of these parts 101, 102, 103, 110 may be integrated. Hence, some of these parts 101, 102, 103, 110 may be integrated and the garment 100 may be manufactured by joining together such separate and/or integrated parts so as to form the complete protective garment 100. For instance, the front part 101 and the back part 102 may be two areas (subparts) of one single integrated part, being joined together with a separate crotch part 110, or the front part 101 and the back part 102 may constitute two separate but joined together parts, the crotch part 110 then forming a third separate but joined together part. These are only a few of many different possible examples of how to construct a protective garment of the present type based on a number of different joined together subparts.

Put differently, the crotch part 110 may be a separate part from said front part 101 and/or from said back part 102. Concretely, the front part 101 and the back part 102 may for instance be manufactured as a tube-shaped textile, after the crotch part 110 joins them together at the groin to form the protective garment 100.

As will be clear from the below, each of the parts 101, 102, 103, 110 may also in itself comprise several separate and joined together parts. For instance, the crotch part 110 may comprise several layers being joined together. Each of such layers may, in turn, overlap or not overlap with the front part 101 and/or back part 102.

The waist 103 may be a separate part, joined together with the front part 101 and the back part 102, or be an integrated subpart of the front part 101 and/or back part 102.

The protective garment 100 may also comprise additional separate or integrated parts, such as the below-described dorsal shaping element 130.

In general, different separate parts of the protective garment 100 may be permanently joined together using one or several joining techniques that may be at least partly conventional as such, such as using stitching, knitting (such as flat knitting), welding and/or gluing.

One example of such joining techniques is bonding. One example of bonding is silicone bonding. Here, silicone is applied, for instance using a screen print process, to a surface of a first layer, such as to form a glue line or a particular desired pattern. Then, a second layer is arranged on top of the first layer, and the two layers are glued together by the silicone, such as by application of pressure and/or heat. The bonding can be performed using a tool having a desired pressing pattern and/or shape to achieve a joint having particular properties, such as a 3D shape in a relaxed state.

Another example is textile welding, whereby heat is applied, such as via hot air or a wedge, to at least two layers arranged on top of each other, welding together the layers using melting along a join line or pattern. As an alternative to heat, ultrasonic energy can be delivered to the welded substrate.

Another example is stitching, that can be conducted using many different types of seams and with different types of one or several threads. For instance, seam types and/or thread types can be selected to achieve local constrictions in a sewn knitted, woven or non-woven material, and/or affect elasticity, stiffness or a shape of the material being stitched. Apart from stitching, both gluing and bonding can also be used to affect 3D properties of the final garment, such as elasticity and stiffness in different directions, and also to impart a certain preformed shape upheld using the glue, bond or seam. These different bonding methods can also be combined in any one produced piece of garment, depending on requirements and desired properties.

In addition, or alternative, to such 3D shaping by bonding, different ones of said parts 101, 102, 103, 110 may also be shaped using seamless techniques, for instance by varying a knitting pattern and/or a knitting needle used and/or a thread used across the surface of such a part 101, 102, 103, 110. In addition thereto or alternatively, the layers forming one or several of said parts 101, 102, 103, 110 may be varied, with respect to material used and/or internal bonds, to achieve similar results.

Similarly, individual ones of said parts 101, 102, 103, 110 and/or separate elements such as the below-described dorsal shaping element 130 or ventral shaping element 140 can be provided with (or arranged in the form of, in the case of said separate elements) lines or patterns of applied silicone or other glues, welds and/or stitches to form features having desired shapes and properties. For instance, such desired properties may include a desired local flexibility, dumbness or elasticity. As a concrete example, the dorsal shaping element 130 may be provided as an application of an elongated silicone area, stitch line or weld line on the back part 102 and/or on the crotch part 110, that may or may not include a separate layer of textile or other flexible material fastened to the back part 102 and/or the crotch part 110 using said silicone, stitch or weld.

Hence, glue, a weld and/or a seam may be applied to a flexible element without joining the element in question to another element, in other words only to affect the local properties of the element in question.

Furthermore, such gluing, stitching and welding can be tailored with specific liquid permeability properties. For instance, a hydrophobic glue or stitching thread, or a liquid-tight weld using completely melted plastic textile material can be arranged to constitute a liquid barrier, preventing liquid from spreading laterally across joined together textile sheets past the joint in question.

More than one of said joining and application techniques may also be combined, such as stitching a glued joint or providing a layer of silicone on top of two stitched together panels of textile material, along a stitch line joining the two panels together. Various joining and applications may also be combined in different layers, having different lateral extensions. For instance, a pattern formed by silicone application may be arranged on top of a layer that is in turn joined to a different layer using stitching, welding or bonding.

As mentioned, surfaces and/or the interior structure of one or several layers of one or several of said parts 101, 102, 103, 110 may furthermore be treated to achieve desired properties without being locally joined at the place of treatment to an additional layer. For instance, fibre flocking can be used, where fibres of plastic material are permanently fastened, such as standing or in random orientations, to the surface in question. Such flocking can be made to achieve a soft surface that is agreeable when in direct contact with the skin of a user and/or affect other mechanical properties such as elasticity or surface friction.

In a different example, molten plastic material can be injected into the layer in question and allowed to set therein, altering the mechanical properties such as elasticity and/or stiffness and/or surface friction of the layer in question. One example of the latter is so-called "liquid lycra" processing.

Another example is the above-discussed surface application of silicone or other compounds, for instance compounds that may in some cases also be used as bonding agents. Such application can locally affect elasticity, stiffness, surface friction, shape and other mechanical properties of the surface to which the application is performed. For instance, silicone can be applied using printing or extrusion, the latter alternative in a process called "silicone extrusion" in which silicone is extruded via a nozzle. Such printed or extruded silicone, that is then present on a surface of the fabric, is capable of producing a 3D shape on the surface of the fabric, can also be flocked with fibres that are fastened to the applied silicone. An additional example is "hidden modulus", where silicone is printed onto a fabric surface so as to penetrate into the material, providing both a desired elasticity along the printed areas and a high-friction surface of the fabric.

In the various examples provided herein, flocked fibres may be fastened using silicone, or any other suitable adhesive, as a binder. Useful flock adhesives include single-part and two-part catalysed systems, and both plastisol and water-based adhesives can be used. Various useful silicone adhesives are described in US2021246312A1. As one of many alternatives, a water-based acrylate coating adhesive is described in CN112794938A.

Seamless knitting, such as tubular knitting or flat-knitting, can also be used to produce parts or all of the present protective garments. In this case, the knitting may be locally varied so as to locally affect mechanical properties, such as elasticity, for instance by introducing local constrictions or similar.

As an alternative to a flocked surface, a velvet material, either elastic or inelastic, can generally be used in the examples described herein.

Hence, various techniques of the above-discussed type can be used to produce the various features described herein, by locally affecting mechanical properties of the different parts 101, 102, 103, 110. The same and/or different techniques can also be used to join various material layers and/or parts 101, 102, 103, 110 together. Sometimes, a joining technique may also affect the mechanical properties in a desired manner, and sometimes a particular joining technique can be combined with a particular application technique as mentioned above to locally achieve a particular set of desired mechanical properties.

The various parts and elements 101, 102, 103, 110, including their individual subparts, of the protective garment 100 described herein may be manufactured with any combination of one or several of the above-described application and/or bonding methods, as the case may be, to achieve the mechanical properties described below in connection to each such element or part.

As mentioned, seamless knitting can be used to produce the protective garment 100. For instance, the whole garment 100, or one or several of said parts 101, 102, 103, 110, can be produced by seamless knitting, without any seams. Then, the garment 100 may comprise various layers as described herein, while at least one such layer is common for all or at least several of the parts 101, 102, 103, 110, while various parts 101, 102, 103, 110 may comprise additional layers. By joining together the various layers, the final garment 100 is then assembled. In a different example, tubular knitting is used in combination with a joining together of the front part 101 to the back part 102 at or via the crotch part 110 to form the final garment 100, with or without additional layers such as described herein.

In particular, in some embodiments at least one layer of the crotch part 110 is manufactured from the same integrated flexible material as at least one layer of said front part 101 and/or at least one layer of said back part 102. A peripheral shape of the said at least one layer of the crotch part 110 is then defined, in relation to said at least one layer of said front part 101 and/or of said back part 102, by areas of varying stitching types, stitching, flocking, gluing and/or welding.

As used herein, the term "integrated material" denotes a body of knitted, woven or non-woven flexible material that has not been joined together from separate subparts, such as using any of the joining techniques described above, but has instead been produced as one connected piece of flexible, knitted, woven or non-woven material by weaving, stitching or some other technique for producing such material. For instance, non-woven materials may be staple non-wovens, melt-blown non-wovens or spunlaid non-wovens. Correspondingly, an "integrated part" is a part being formed as a part of the same, connected material body as it is a part of.

As the term is used herein, a "non-woven" material is a fabric-like material made from short and/or long fibres that are bonded together, such by chemical, mechanical, heat or solvent treatment. One example of such material is felt. A non-woven material is neither woven nor knitted.

The waist 103 may comprise a waist band, which may in turn be formed with desired elastic and friction properties by application of silicone to the surface of the waist near and/or at an upper edge of the waist 103. This silicone can also form a protection against fraying. The corresponding technique can be used to form leg opening edges of the garment 100.

The description herein uses a standing user wearing the protective garment 100 as a frame of reference for the various parts and directions of the garment 100. Hence, the geometry of the user's anatomy (for instance, "dorsal" and "ventral") and/or an external coordinate system (such "up" or "down") are used to characterise various geometric aspects.

As will be understood from the following, the user is typically a female user. However, it is understood that the present principles can also be applied for garments intended for male users, as applicable.

According to the invention, the crotch part 110 comprises a leak-proof body 120. In many cases, the leak-proof body 120 in turn comprises or is constituted by a liquid-absorbing layer 124, arranged to absorb and hold liquid released onto the leak-proof body, such as a user wearing the protective garment 100. Typically, the leak-proof body 120 will furthermore comprise a liquid barrier 126, such as a plastic film, arranged to prevent leakage of liquid through the leak-proof body 120 and out from the protective garment 100 when worn by a user. In some cases, the leak-proof body 120 may contain no liquid-absorbing layer, but provide its leak-proofness only via a liquid barrier 126. The liquid barrier 126 in itself may be in the form of or comprise a flexible fabric having a liquid-proof-treated coating or surface; a liquid-impermeable (such as hydrophobic and/or impregnated) fabric and/or a plastic film. In specific examples, the liquid barrier 126 may comprise a knitted, wowen or non-woven material which it in itself hydrophobic, such as a so-called spunbond-meltblown material, comprising one or several meltblown layers sandwiched between a pair of spun-bonded layers. In other words, the liquid barrier 126 may be arranged to be penetrable to water vapour (to "breathe"), but at the same time be impermeable to liquids. Of course, the liquid barrier 126 may in itself comprise several layers, each having one or several of said different properties.

According to one aspect of the present invention illustrated in Figures 1a-1e, the protective garment 100 comprises a dorsal shaping element 130.

As used herein, the term "shaping element" refers to a separate or integrated part of the garment 100 arranged to locally affect the shape of the garment when a standing user wears the protective garment 100, and in particular as a result of a shaping of the garment 100 being a consequence of said wearing of the garment 100 (as opposed to when the garment 100 is not worn but instead, for instance, lays freely on a surface). In other words, the presence of the shaping element alters the shape of the protective garment 100 as it is worn by the standing user in a way so that the shape is different, and in particular functionally different, as compared to the hypothetical case in which the garment 100 without the shaping element in question.

The dorsal shaping element 130 is arranged along the intergluteal cleft of the standing user wearing the protective garment 100. It is fastened to or comprises an integrated part of the leak-proof body 120, and is fastened to or comprises an integrated part of the back part 102.

In the example illustrated in Figure 1a, the dorsal shaping element 130 comprises a seam interconnecting the back part 102 and the crotch part 110, while extending some ways dorsally from a dorsal end edge 110a of the crotch part 110 along the back part 102. Hence, in this example the dorsal shaping element 130 constitutes a separate part (the seam itself), while interconnecting the back part 102 and the crotch part 110. It is realized that the back part 102 and the crotch part 110 may also be connected in additional ways, such as along a lateral joining line that may not be part of the dorsal shaping element 130. Such joining may involve one or several layers of the back part 102 and/or the crotch part 110. The seam may be sewn to create a local constriction of the fabric, and the stitching technique and/or thread may be selected so that the constriction is arranged to locally provide an increased elasticity and/or stiffness of the fabric.

In general, the dorsal shaping element 130 may comprise a part 132 being separate from (but permanently fastened to) the crotch part 110 and the back part 102, the separate part 132 connecting the crotch part 110 to the back part 102.

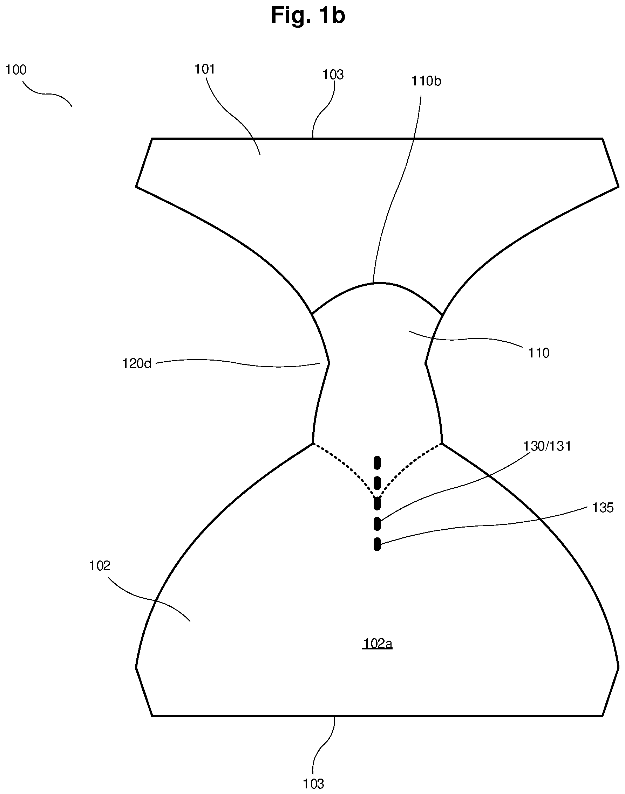

In the example illustrated in Figure 1b, the dorsal shaping element 130 instead comprises an elongated constriction in the seamless-stitched material of the garment 100, in an embodiment where the back part 102 and the crotch part 110 are integrated parts in relation to each other. The dorsal shaping element 130 runs across both the back part 102 and the crotch part 110, and the constriction pulls together the fabric locally so as to achieve a locally increased tension in the fabric when the fabric is pulled along its surface, in particular when it is pulled apart in a dorsal-anterior direction. The constriction may also be arranged to locally provide an increased elasticity and/or stiffness of the fabric.

Generally, the dorsal shaping element 130 may comprise an integrated part 131, that may constitute the entire shaping element 130 or only a part of the shaping element 130 then further comprising additional parts, of a knitted or weaved textile layer of the protective garment 100. Such a knitted or weaved textile level is then produced by, during manufacturing, locally varying the properties of said textile layer by changing the weaving or knitting procedure, as discussed above.

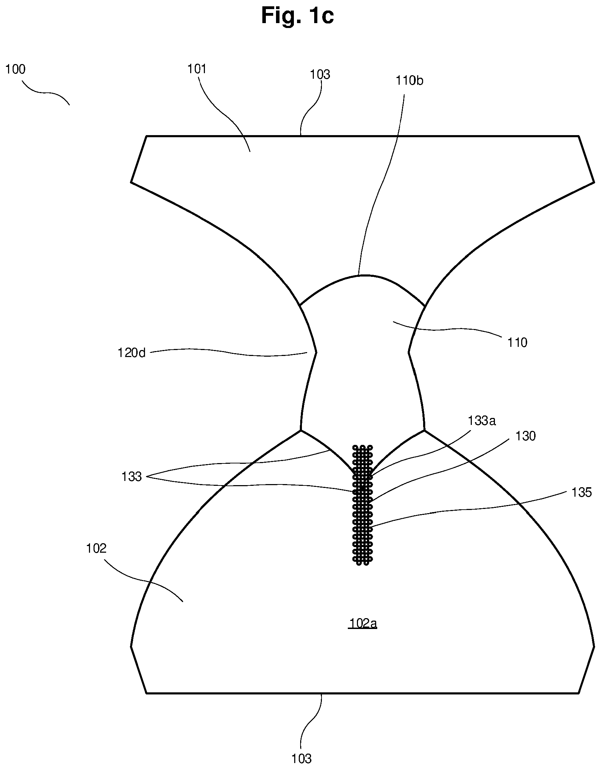

In the example illustrated in Figure 1c, back part 102 is joined to the crotch part 110 using a seam or a glue/weld bond, while the dorsal shaping element 130 is applied in the form of a patterned elongated silicone application on the surface of a respective layer of the back part 102, also extending across the surface of a layer of the crotch part 110. The silicone may be arranged to locally provide an increased elasticity and/or stiffness of the fabric, and may be arranged on an inner surface or on an inner layer so that it is not visible externally on the protective garment 110. The dorsal shaping element 130 may comprise a flocked area, with corresponding properties. This flocked area may be a part of a larger set of flocked areas of the different types described herein, such as fold lines 129a and/or the "Y"-shape and/or the ventral shaping element 140 discussed below. In particular, such a flocked dorsal shaping element 130 may extend outside of the crotch part 110 and/or outside of the leak-proof body 120, along the back part 102. The corresponding also applies to the ventral shaping element 140 with respect to the crotch part 110, the leak-proof body 120 and the front part 101.

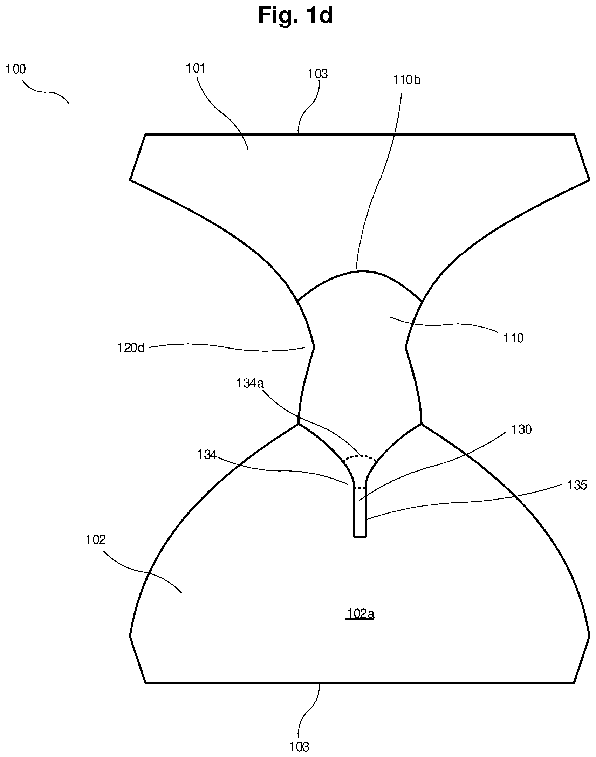

In the example illustrated in Figure 1d, the crotch part 110 is shaped with a dorsally elongated extension, overlapping with the back part 102, the elongated extension being fastened to the back part 102 by a suitable seam or glue/weld joint and constituting the dorsal shaping element 130. The overlap and/or the joining itself is arranged to locally provide an increased elasticity and/or stiffness of the fabric. It is understood that the dorsally elongated extension may comprise all layers of the crotch part 110 or of the leak-proof body 120, or only some of said layers, such as the liquid-absorbing layer 124 and/or the liquid barrier layer 126 (see below).

As have been made clear above with respect to combinations in general, the various examples illustrated in Figures 1a-1d may be combined in different ways.

Moreover, the back part 102 comprises a panel part 102a, separating the shaping element 130 from the waist 103, such as an upper end edge of the waist 103, so that the dorsal shaping element 130 does not extend all the way up the waist 103 (or the upper end edge of the waist 103) of the protective garment 100 worn by the standing user. The panel part 102a is preferably entirely made of flexible, non-stiff knitted, woven or non-woven material. The dorsal shaping element 130 may be separated from the waist 103 or said end edge of the waist 103 by at least 2 cm, such as at least 5 cm, depending on the model and size of the garment 100.

It is understood that, in case the garment 100 comprises an upper or torso part, there may be no end edge of the waist 103. It is, however, preferred that even in this case the waist 103 is defined as an elastic part running around the waist of the user wearing the garment 100.

Namely, the dorsal shaping element 130 is arranged so that, when a dorsal/upward force is applied to the dorsal shaping element 130 via the panel part 102a of the back panel 102 as a result of the waist 103 being pulled upwards in relation to said user wearing the protective garment 100, it in turn forces a part, such as a laterally central part, of the crotch part 110 extending along the intergluteal cleft of said user in an upwards direction.

By forcing the crotch part 110 upwards in relation to the standing user wearing the protective garment 100, the crotch part 110 is pressed into the intergluteal cleft of the user, providing a tighter fit.

This is illustrated in Figure 1e, where F denotes a fold line along the intergluteal cleft of the user wearing the protective garment 100, the fold line F arising as a result of said forcing upwards of the crotch part 110 in relation to the user.

In particular, it is preferred that the leak-proof body 120, such as said liquid-absorbing layer 124, is pressed into the intergluteal cleft of the user this way.

Hence, said upwards forcing may also, to more or less extent, have a anteriorly forcing component in relation to said standing user wearing the protective garment 100, at least with respect to a part of the crotch part 110 in the vicinity of the dorsal shaping element 130.

As a result of this pressing in, the crotch part 110, and in particular said leak-proof body 120, such as said liquid-absorbing layer 124, is deformed so as to form a V-shape locally, at least in the vicinity of the dorsal shaping element 130, following the shape of the intergluteal cleft of the user. This is illustrated in Figure 2, where the user is denoted U and the intergluteal cleft of the user is denoted IGC.

The pressing in is generally achieved by the panel part 102a pulling on the dorsal shaping element 130, and the dorsal shaping element 130, by it being elastic or inelastic, and/or stiff, does not deform/stretch (along a dorsal-ventral line) as much as the fabric of the back part 102 (and possibly also the crotch part 110) surrounding the dorsal shaping element 130, resulting in a locally applied dorsally-ventrally directed tensile force, in turn forcing the crotch part 110 inwards into the intergluteal cleft of the user as described. It is realized that, in the case in which the dorsal shaping element 130 is elastic, it is arranged to stretch as a result of said pulling upwards of the waist 103, but to a lesser extent than the surrounding back part 102 material, resulting in a bulging inwards of the garment 100, into the intergluteal cleft in the way described. In some embodiments, the dorsal shaping element 130 may be prestressed, such as by provided with local fabric wrinkles in combination with an elastic elongated structure urging the dorsal shaping element 130 to compress in the ventral-dorsal direction in the sagittal plane of the standing user wearing the protective garment, or by providing the fabric of the protective garment 100 in the local vicinity to the dorsal shaping element 130, as well as the dorsal shaping element 130 itself, as an elastic fabric, achieving the corresponding effect.

It is noted that the dorsal shaping element 130 is arranged to provide properties of the protective garment 100 in the immediate vicinity of the dorsal shaping element 130 that differ from properties of the protective garment 100 at a distance from the dorsal shaping element 130 in order to achieve that the dorsal shaping element is forced upwards-inwards when the waist 103 is pulled upwards as described herein.

The present inventor has discovered that such a dorsal shaping element is effective in achieving a close fit of such a protective garment 100, in particular in case it is not connected to the waist 103 as described above, providing a very tight fit (and therefore also leak-protection) while still achieving a garment with great comfort. Since the dorsal shaping element 130 does not extend all the way up to the waist 103, design freedom also greatly increases. When worn, the dorsal shaping element 130 may even be more or less non-visible.

Hence, the dorsal shaping element 130 is arranged so that an upwards pulling force applied to the waist is translated, via the panel part 102a, to an upwards/ventral pulling force applied to the dorsal shaping element 130, in turn pressing the crotch part 110 into the intergluteal cleft of the user wearing the garment 100, also forcing at least part of the crotch part 110, and in particular the leak-proof body 120, to deform, such as into a saddle shape, following the local curvature of the skin of said user.

To accomplish this, the dorsal shaping element 130 must be connected to the crotch part 110 in some way. To this end, the dorsal shaping element 130 may constitute, be comprised in or comprise a joining area 133, joining together the leak-proof body 120, such as a liquid-absorbing layer 124 and/or a liquid barrier layer 126 comprised in the leak-proof body 120, to the back part 102.

One example of this is that of Figure 1a, wherein said seam forms part of such joining area 133 in that it connects the leak-proof body 120 to the back part 102. It is understood that a lateral joint between the leak-proof body 120 and the back part 102 would also constitute parts of such joining area 133, as is illustrated in Figure 1a.

Similarly, the silicone application illustrated in Figure 1c can extend onto the leak-proof body 120, and hence be a part of the joining area 133.

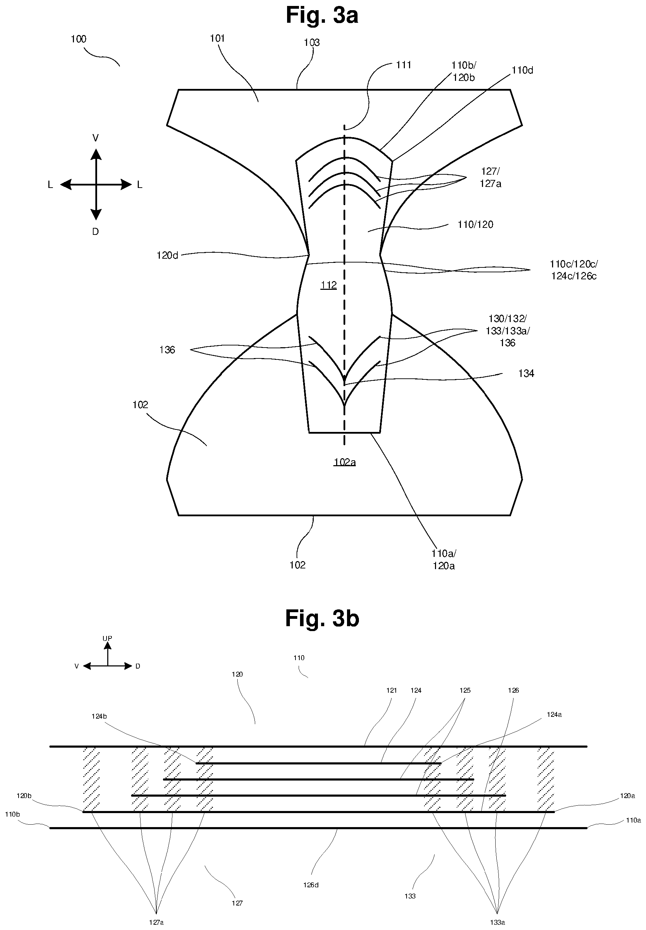

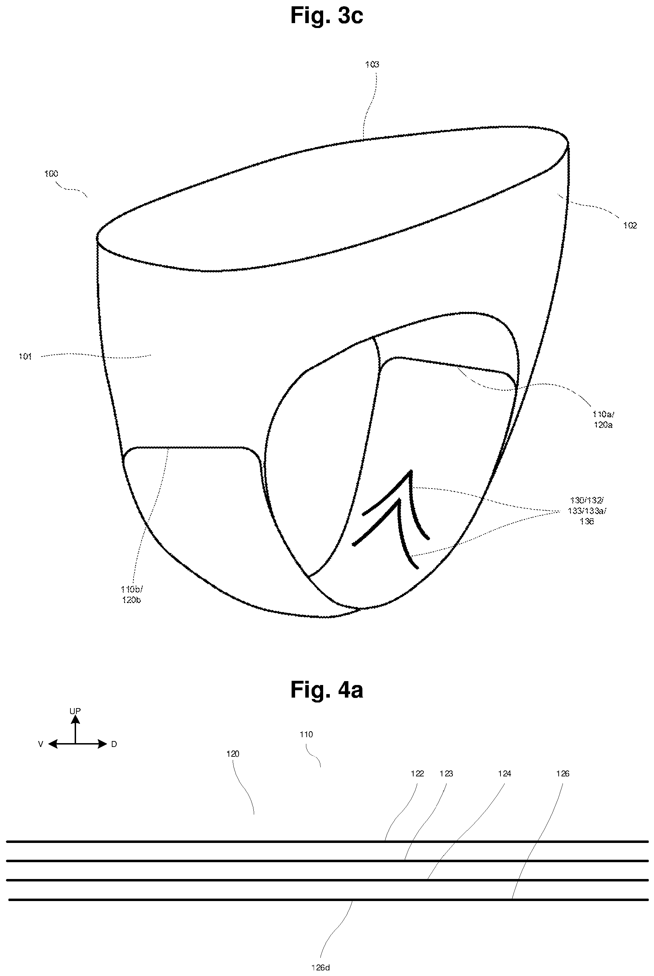

In particular, the dorsal shaping element 130 may comprise joining means 133a, such as a stitched joint, glue joint or weld joint. Such joining means 133a may then join the leak-proof body 120 to the back part 102. In the examples of Figures 1a and 1c, the joining means 133a constitute (comprise) the seam and the silicone application, respectively, at the juncture between the back part 102 and the leak-proof body 120 of the crotch part 110. Another example is illustrated in Figures 3a-3c, see below.

Even though it is possible to apply the above principles in a disposable, one-time use garment, in preferred embodiments the protective garment 100 is a washable garment arranged for repeated use. This means that all material constituting the garment 100 described herein is preferably wettable and dryable in a reversible fashion, and all material is preferably permanently fastened rather than loosely arranged inside compartments or similar.

As illustrated in Figures 1a-1e, the dorsal shaping element 130 extends dorsally and upwards along the intergluteal cleft of the standing user wearing the protective garment 100.

As seen in the examples illustrated in Figures 1a-1e, it is preferred that the dorsal shaping element 130 comprises a part extending dorsally-ventrally along the intergluteal cleft of the standing user wearing the garment 100. Even if this is not strictly necessary, it is preferred that such an extended part is connected (in contrast to the case in which it is divided into more than one disjoint subparts). Preferably, the dorsal shaping element 130 as a whole, at least a connected part of the dorsal shaping element 130, and/or in certain embodiments said part extending along the intergluteal cleft, has a length of at least 3 cm running along the intergluteal cleft of the standing user wearing the protective garment 100. It is understood that the dorsal shaping element 130 can have other shapes, as will be exemplified below, and also that the dorsal shaping element 130 can comprise additional shapes and parts, such as shapes or parts extending laterally rather than dorsally.

In all these cases, the dorsal shaping element 130 provides, when the garment 100 is worn and in particular as the waist 103 is pulled upwards, an increased dorsal-ventral tension in the material of the garment 100 along the intergluteal cleft (as compared to a dorsal-ventral tension in other parts of the garment 100) of the user wearing the garment 100, pressing the dorsal shaping element 130 and as a result the crotch part 110 upwards-inwards into the intergluteal cleft.

In addition to the dorsal shaping element 130, in some embodiments the protective garment 100 further comprises a pair of elongated ventral shaping elements 140, illustrated in Figure 1a.

The ventral shaping elements 140 may be provided in one of the ways described above, i.e. as a surface-deposited pattern or line of silicone or other adhesive; as a seam with particular properties; as a flocked pattern or line; as a joint between two layers; etc. It is arranged to extend on either lateral side of the labia of said standing user wearing the protective garment 100, ventrally from the dorsal shaping element 130. In other words, there may be at least two elongated ventral shaping elements 140, each extending on a respective lateral side of said labia, each such elongated ventral shaping element achieving an increased tension in the material of the garment 130 along the (generally ventral-dorsal) extension line of each such ventral shaping element 140, locally forcing the garment 100 upwards in relation to the standing user wearing the garment 100. In particular, the crotch part 110, such as the leak-proof body 120, such as the liquid-absorbing layer 124, and in particular lateral side portions surrounding the labia of the user, is pressed upwards this way.

In particular, the ventral shaping elements 140 may be arranged so that, when a ventral/upward force is applied to the ventral shaping elements 140 via the front part 101 as a result of the waist 103 being pulled upwards in relation to said user wearing the protective garment 100, it is in turn forced upwards.

Since the ventral shaping elements 140 force the garment upwards on either side of the labia, but not across the labia, a very snug fit is achieved while not compromising comfort. This is particularly the case when combining the ventral shaping elements 140 with the dorsal shaping element 130 of the above-described type, since the combination will provide a snug fit around the whole genitals of the female user. This is particularly true in the preferred case in which the dorsal shaping element 130 is directly connected to the ventral shaping elements 140, such as is illustrated in Figure 1a. This can advantageously be achieved by arranging the dorsal shaping element 130 together with said ventral shaping elements 140 to together form a "Y"-shape, as can be seen in Figure 1a. Such a combination of the dorsal shaping element 130 and the ventral shaping elements 140 will distribute the above-discussed increased tension forces along the whole line from the user's intergluteal cleft to a respective point along or ventrally past the labia of the user, efficiently sealing the crotch part 110, and in particular the leak-proof body 120, against the skin of the user. It is understood that such a configuration will provide a forced saddle-shape to leak-proof body 120.

As is also illustrated in Figure 1a (see also Figures 3a and 3b), said ventral shaping elements 140 connect to either side of a ventral end edge 110b of the crotch part 110. In addition or as an alternative thereto, and as is illustrated in Figure 1a, the ventral shaping elements 140 may connect to a respective side edge 110c of the crotch part 110, such as to a ventral end point 110d of such a lateral side edge 110c. Correspondingly, the connection may also or instead be to a ventral end edge 120b and/or such a ventral side edge 120c, such as to a ventral end point of such a side edge 120c, of the leak-proof body 120 and/or to a ventral end edge 124b and/or side edge 124c, such as to a ventral end point of such a side edge 124c, of a liquid-absorbing layer 124 comprised in the leak-proof body 120. This provides a good liquid seal to the parts of the crotch part 110 intended to prevent liquids from leaking out from the garment 100.

As is also illustrated in Figures 1a-1e (see also Figures 3a-3b), the crotch part 110, the leak-proof body 120 and/or the liquid-absorbing layer 124 comprised in the crotch part 110 and more particularly comprised in the leak-proof body 120, may comprise a respective ventral end edge 110b; 120b; 124b, generally laterally elongated and having a convex shape softly bulging ventrally/upwards in relation to said standing user wearing the protective garment 100.

Such a bulge provides improved comfort and fit, while not jeopardizing leak-proof security. The present inventor has discovered that said bulge of the convex shape provides particularly good fit if extending at least 3 cm, and optionally at the most about 40 cm, such as at the most 30 cm, such as at the most 20 cm, 10 cm, ventrally/upwards, along the surface of the garment 100, in relation to said standing user wearing the protective garment 100.

As is furthermore illustrated in Figures 1a-1e (see also Figures 3a-3b), the crotch part 110, the leak-proof body 120 and/or the liquid-absorbing layer 124 also comprises a respective generally laterally elongated dorsal end edge 110a; 120a; 124a. This dorsal end edge 110a, 120a, 124a may then comprise a direction change 134, at the intergluteal cleft of said standing user wearing the protective garment 100, the direction change 134 defining an acute angle 134a pointing upwards/dorsally in relation to the user.

The dorsal shaping element 130 can comprise a straight part 135 extending dorsally and upwards from the leak-proof body 120. In particular, such a straight part may advantageously extend at least 1 cm, such as at least 2 cm, such as at least 3 cm, dorsally from the apex of said acute angle 134a. Furthermore, the straight part may extend at the most 30 cm, such as at the most 20 cm, such as at the most 15cm, dorsally from the apex of said acute angle 134a.

An overlap between the dorsal shaping element 130, such as said straight part 135, and at least one of the crotch part 110 and the leak-proof body 120 may advantageously be between 1-30, such as 1-20 cm, of length.

As is seen in Figures 1a-1e (see also Figures 3a and 3b), the above-mentioned joining means 133a may furthermore be arranged at said dorsal end edge 124a of the liquid-absorbing layer 124 of the leak-proof body 120.

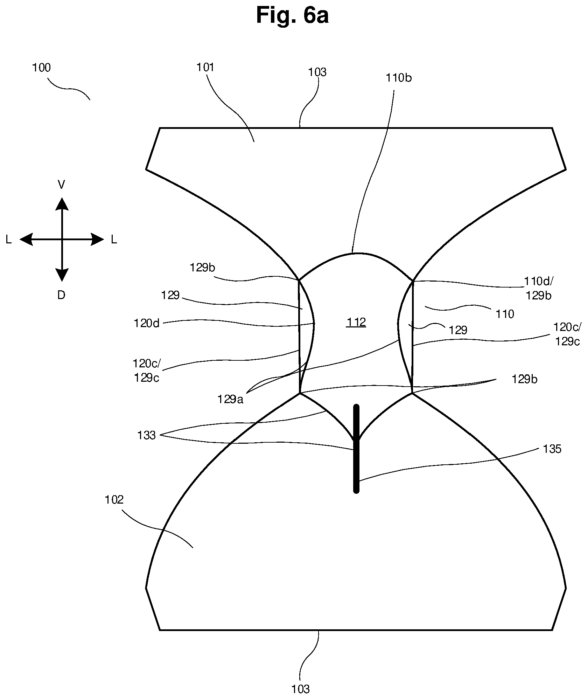

As is also illustrated in Figures 1a-1e, the leak-proof body 120 and/or a central part of the leak-proof body 120 being defined between a pair of dorsally-ventrally extending fold lines 129a in the leak-proof body 120 (see Figures 6a-6b, below) has a laterally local narrowest part 120d arranged between the legs, such as at or near the gracilis in a ventral/dorsal direction, of the standing user wearing the protective garment 100.

Then, as is illustrated in Figures 1a-1e, said side edges 120c; 124c of the leak-proof body 120 and/or of the liquid-absorbing layer 124, and/or of said central part of the leak-proof body 120, may diverge laterally outwards from said laterally narrowest part 120d, preferably in both dorsal and ventral directions. This provides a very good fit, comfort and leak-proofness, in particular in combination with the dorsal 130 and/or ventral 140 shaping elements of the types described herein.

In particular, it has proven advantageous that said side edges 120c; 124c then both extend between the dorsal end edge 124a of the liquid-absorbing layer 124 comprised in the leak-proof body 120 and the ventral end edge 124b of the liquid-absorbing layer 124. The side edges 120c, 124c may then be pulled ventrally/upwards by ventral shaping element 140, while a midpoint at the dorsal end edge 120a, 124a is pulled dorsally/upwards by the dorsal shaping element 130, very efficiently achieving said saddle-shape of the leak-proof body 120.

Figures 3a-3c illustrate another exemplifying protective garment 100. In particular, Figure 3b shows, in a cross-section as seen from the side, various layers that may be comprised in the crotch part 110.

As seen in Figure 3a, the crotch part 110 may extend along a thought path 111 along the sagittal plane of the standing user wearing the protective garment 100, at least part of said path 111 extending along the intergluteal cleft of said standing user.

Furthermore, as illustrated in Figure 3b, the crotch part 110 may comprise, in order from an inner part of the crotch part 110 to an outer part of the crotch part 110:
First, a flexible first layer 121. This layer 121 may be an inner and/or external layer, arranged to directly contact the skin of the user wearing the garment 100. For instance, this inner layer may be a knitted, woven or non-woven material, such as a textile material or other fabric.

Thereafter, a flexible second liquid-absorbing material layer 124 extending along said path 111. The second layer 124 may be the same as the liquid-absorbing layer 124 discussed above (and below), and may comprise a material capable of absorbing and holding a liquid.

Thereafter, a flexible third liquid barrier layer 126. This third layer may be an outer external layer, forming an external surface of the garment 100. However, the garment 100 may also comprise a flexible outer external layer 126d, that may comprise any suitable textile material and form part of the crotch part 110 or constitute a separate part of its own.

It is realized that the crotch part 110 may additionally comprise any other layers, depending on the detailed application.

As used herein, the terms "inner" and "outer" generally refer to a relative position in relation to a user wearing the garment 100, such that "inner" arranged parts are closer to the skin of the user than "outer" arranged parts when the garment 100 is worn.

In contrast thereto, "internal" and "external" generally refer to a relative position in relation to the garment 100, such that "internal" parts are located within the garment 100 whereas "external" parts are located on its outside surface.

As is illustrated in Figure 3b, the respective extensions of the crotch part 110, the leak-proof part 120 and the liquid-absorbing part 124 (if present) can vary.

In general terms, the crotch part 110 is a part arranged at the crotch of the user wearing the protective garment 100.

Also on a general note, the leak-proof part 120 is a part arranged to block, when the garment 100 is worn by the user, liquid leakage by being liquid impermeable and/or absorbing.

Also generally, the liquid-absorbing 124 is a part arranged to absorb, when the garment 100 is worn by the user, such blocked liquid.

In various embodiments, there may however be various types of dorsal/ventral/lateral overlap between the constituent subparts thereof, for instance as exemplified herein. There may also be various types of dorsal/ventral/lateral overlap between any one or several of the various subparts of the crotch part 110 (including the leak-proof part 120, the liquid-absorbing layer 124, the liquid barrier layer 126, and so forth) and other parts of the garment 100 (including the front part 101 and the back part 102).

For instance, and as is illustrated in Figure 3b, the liquid barrier layer 126 may extend further along said path 111 than one, several or all of any liquid-absorbing layers 124, 125, so that the liquid barrier layer 126 extends along the path 111 along the entire length of the extension along said path 111 of the liquid-absorbing layer 124 and also along at least a part of the path 111 not occupied by the second layer 124 dorsally in said sagittal plane of the user. In other words, the liquid barrier layer 126 may extend further in front and/or in the back than the liquid-absorbing layer 124.

As is also clear from Figures 3a-3c, the crotch part 110 may comprise a dorsal joining area 133, joining together the inner layer 121 and one or several of said liquid-absorbing layer(s) 124. Moreover, such a dorsal joining area 133 may extend laterally in relation to the standing user wearing the protective garment 100, fastening the one or several absorbing layer(s) 124 to the inner layer 121. As mentioned above, the layer 121 may be an interior layer, but it may also be a non-interior layer, then preferably arranged closed to the skin of the wearing user than the liquid-absorbing layer(s) 124.

In the example illustrated in Figures 3a-3c, it can be seen that the dorsal joining area 133 comprises two generally laterally elongated shapes 136, each comprising a direction change 134 of the above-discussed type, arranged, at (along) the intergluteal cleft of said user and defining a respective acute angle 134a pointing upwards/dorsally in relation to the user. It is realized that there may be one or more than one, such as three or more, such laterally elongated shapes comprised in said dorsal joining area 133, and that one or several of such lateral shapes may comprise such a direction change 134. It is understood that said laterally elongated shapes 136 may constitute joining means 133a of the above-discussed type.

As a matter of fact, said laterally extending shape 136 having said direction change 134 may constitute the dorsal shaping element 130, having the effect described above, forcing the leak-proof body to assume the shape of the user in the area of the intergluteal cleft. More generally, the dorsal joining area 133 may in fact comprise or itself constitute the dorsal shaping element 130 in question. In the example illustrated in Figures 3a-3c, each of the two laterally extending shapes having an acute-angle direction change 134 constitutes a dorsal shaping element 130 of its own right, located at slightly different dorsal locations along the user's intergluteal cleft, at least for such shaping elements interconnecting a part of the crotch part 110 to the back part 102.

As mentioned above, the various embodiments disclosed can be combined in any manner, subject to compatibility. One such example is that a joining area 133 of the type illustrated in Figures 3a-3c can be combined with a straight part 135 of the dorsal element such as the one illustrated in Figures 1a-1e. Such straight part 135 can then be, for instance, applied on an interior or non-interior surface of the crotch part 110 and/or leak-proof body 120, or be arranged in the form of a joining line joining together two or more of said layers 121, 124, 125, 126.

As also discussed above, the liquid-absorbing layer 124 may have a laterally extending dorsal end edge 124a. In the example shown in Figures 3a-3c, the dorsal joining area 133 fastens the liquid-absorbing layer 124 to the inner layer 121 along this dorsal end edge 124a.

More particularly, as shown in said Figures and perhaps most clearly in Figure 3b, the different dorsal joining means 133a are arranged to fasten different ones of several liquid-absorbing layers 124-125 to the inner layer 121, and optionally also to the liquid barrier layer 126.

Hence, the dorsal joining area 133 may comprise several generally laterally elongated and possibly separated shapes 136 of said type, distributed along the intergluteal cleft of the wearing user, whereby at least two of said several elongated shapes 136 at least partly may overlap with the liquid-absorbing layer 124, or with at least one of said liquid-absorbing layers 124, 125 in case there are several liquid-absorbing layers. in that case, the crotch part 110 may comprise several liquid-absorbing layers 124, 125, where different ones of said shapes 136 of the dorsal joining area 133 join together different sets of said liquid-absorbing layers 124, 125 to the inner layer 121.

In Figure 3b it is shown how each (or at least one or some) of the one or several liquid-absorbing layers 124, 125 have a respective dorsal end edge 124a (in Figure 3b, only the dorsal end edge 124a for layer 124 is shown), running along (or possibly dorsally of) a respective fastening line of a respective elongated shaping element 136, joining the liquid-absorbing layer 124, 125 in question to the inner layer 121.

More generally put, a more dorsally arranged, in relation to the standing user wearing the protective garment 100, one of said ventrally elongated shapes 136 of the dorsal joining area 133 may be arranged to join together a smaller number of said liquid-absorbing layers 124, 125 than a more ventrally arranged one of said shapes 136.

In Figure 3b, the liquid-absorbing layers 124, 125 are arranged so that dorsally further extending liquid-absorbing layers are arranged closer to the user's skin. However, depending on the concrete embodiment, dorsally further extending liquid-absorbing layers 124, 125 may instead be arranged further from the user's skin.

This provides a very controlled absorption of liquid otherwise risking escaping in a dorsal direction, while maintaining high comfort. The absorption of the liquid can be focussed at and around a central area 112 of the crotch part 110, while the absorption capacity is gradually decreased further away from said central area 112.

In many applications, one, several or all of the joining means 133a may constitute a ventral, dorsal and/or lateral liquid barrier. For instance, the joining means 133a in question may be manufactured as a seam using a hydrophobic thread; or as a deep and full weld of joined together plastic material of the layers in question. The dorsal joining area 133 may also comprise liquid barriers arranged otherwise, arranged to restrict flow of liquid in a lateral/dorsal/ventral direction along the leak-proof body 120, with the aim of allowing liquid to be distributed across one or several liquid-absorbing layers 124, 125 but not escape outside of an area being covered by such liquid-absorbing layers 124, 125.

One important aspect of the general embodiment exemplified in Figures 3a-3c is that the liquid barrier layer 126 must be kept intact, so as to remain liquid-proof. Hence, in case the dorsal joining area 133 comprises stitches joining together said layers 121, 124, 125, such stitches will not penetrate through the third layer 126. If a joining means 133a is instead arranged in the form of a silicone bonding, the silicone can be applied directly to the liquid barrier layer 126 so as to fasten the liquid barrier layer 126 to one or more of the liquid-absorbing layers 124, 125 and/or to the inner layer 121.

In some embodiments, one or several of said liquid-absorbing layers 124, 125 and the liquid-absorbing layer 126 layer may be fastened together along respective lateral side edges 124c, 126c of said layers. Apart from such a possible joint, it is preferred that the liquid barrier layer 126 can move freely, in a lateral/dorsal/ventral direction, in relation to the one or several liquid-absorbing layers 124, 125, at least in said central area 112 of the crotch part 110, the central area 112 being located between the dorsal joining area 133 comprising shapes 133a and the below-described ventral joining area 127 comprising shapes 127a. The present inventor has realized that this provides superior comfort while not jeopardizing leakage security.

In order to increase leakage security, in some embodiments the liquid barrier layer 126 is arranged to extend, in a dorsal-ventral direction, at least 25 cm along said path 111 for a medium sized garment according to EN 13402, European standard for Size designation of clothes. Such a medium size corresponds to a waist girth of 76-80 cm. At the same time, one, several or all of the liquid-absorbing layers 124, 125 may not extend as far, so that the layer 126 forms an additionally extended leakage-securing layer extending past the extent of any liquid-absorbing layers 124, 125. This way, the bulkiness of the garment 100 can be decreased towards the sides of said central area 112, while still providing adequate leakage protection.

In a way similar to the dorsal joining area 133, Figures 3a-3c also show that the crotch area 110 may comprise a ventral joining area 127, joining together the inner layer 121 and the one or several liquid-absorbing layers 124, 125. in a way corresponding to the dorsal joining area 133, the ventral joining area 127 also extends laterally in relation to said standing user wearing the protective garment 100, and is arranged to fasten one, several or all of the liquid-absorbing layers 124, 125 to the inner layer 121.

The ventral joining area 127 may furthermore be arranged to fasten the one or several liquid-absorbing layers 124 to the inner layer 121 along a ventral end edge 124b of one or several liquid-absorbing layers 124, 125, in a way corresponding to what has been said in relation to the dorsal joining area 133. The ventral joining area 127 may also constitute a liquid barrier, in the above-discussed manner.

Moreover, the ventral joining area 127 may comprise a generally laterally elongated shape having a convex shape softly bulging ventrally/upwards in relation to said standing user wearing the protective garment 100. The function of this convex shape may be as described above in relation to the ventral end edge 110b of the crotch part 110.

In particular, and as is illustrated in Figures 3a-3c, the ventral joining area 127 may comprise several such generally laterally elongated shapes 127a, distributed along a thought line between the perineum and the bellybutton of said standing user wearing the protective garment 100. At least two of said several elongated shapes 127a may at least partly overlap with one, several or all of said one or several liquid-absorbing layers 124, 125.

In particular, and in a way corresponding to what has been said with respect to the dorsal joining area 133, different ones of said ventral shapes 127a of the ventral joining area 127 may join together different sets of said liquid-absorbing layers 124, 125 to the inner layer 121. For instance, a more ventrally arranged, in relation to said wearing user, one of said ventral shapes 127a may be arranged to join together a smaller number of said liquid-absorbing layers 124, 125 than a more dorsally arranged one of said ventral shapes 127a.

As mentioned above, each of the liquid-absorbing layers 124, 125 is arranged to absorb a liquid, preferably in a manner so that it can hold the liquid without releasing it even if subjected to mild pressure. In contrast thereto, a spacer or wicking material can hold a liquid but, like a sponge, will again release the liquid if pressed upon, sometimes even at low pressures. Hence, the liquid-absorbing layers 124, 125 may preferably bind the liquid more strongly, for instance using a chemical bond. In preferred embodiments, the one or several liquid-absorbing layers 124, 125 comprise so-called "super-absorbing" materials, such as a super-absorbing polymer ("SAP") material, forming a hydrogel when exposed to water. Preferably, such super-absorbing material is arranged in the form of a super-absorbing fibre material. In washable applications, the fibres may be manufactured from a material that does not dissolve but maintains structural integrity when exposed to water and as a result swells, such fibres being permanently bound to a flexible substrate of the liquid-absorbing layer 124, 125 in question. Super-absorbing fibers useful for the present purposes may also be contained inside a fabric container, such as a permeable mesh barrier. For instance, known super-absorbing materials comprise water absorbent, water insoluble, polymeric materials manufactured by polymerizing a water soluble monomer or monomer blend, such as acrylic acid, in the presence of a polyethylenically unsaturated monomer, such as N,N'methylenebisacrylamide. See, for instance, US 2011172621 A1 for more information about super-absorbing materials.

When such fibre materials are washed and dried, they release any absorbed bodily fluids and return to a state in which they are again prepared to absorb liquids anew.

Such super-absorbing fibre containing material may be a non-woven material.

However, in addition to such a liquid-absorbing layers 124, 125, the crotch part 110 may also comprise one or several distribution layers 122, 123 (see Figures 4a-4e). Such distribution layers are arranged to allow liquid entering into the layer in question to distribute in a defined way, with the intention of making the liquid in question reach a particular liquid-absorbing layer 124, 125 for absorption.

In general, such distribution layers may be made from so-called wicking materials, such as so-called spacer materials/spacer fabrics. This is a material that has been manufactured to contain voids, forming an open-pore structure that absorbs, holds and transports liquids through capillary forces, much like a sponge. For instance, such spacer materials can be made as stitched textiles. By controlling the stitching in 3D space, such spacer material may be manufactured to display open-pore structures having different properties.

In one example, the open-pore structure may be arranged to transport, by capillary forces, liquids more efficiently along a first material axis as compared to along a second material axis. This can be achieved by, for instance, providing more densely distributed pore walls along the first material axis than along the second material axis, or by providing hydrophobic threads running along the first material axis but not along the second material axis, in a spacer material otherwise manufactured from hydrophilic threads.

For instance, the spacer material may be designed this way to transport, by capillary forces, liquids more efficiently along a surface of the material (inside the material in question) as compared to perpendicularly through the material in question, or vice versa.

It is also possible to manufacture spacer material transporting liquids in any direction through the material more or less equally efficiently.

Moreover, such distribution material may be manufactured with larger of smaller pore sizes, resulting in that held liquid will be transported, by capillary forces, more or less efficiently irrespective of direction.

Distribution materials of said types may also be denoted wicking materials, and hence a "distribution layer" may also be called a "wicking layer". Such a distribution layer may also serve as an entry layer, being arranged with an open surface to allow liquid to enter into the distribution layer in question and then be distributed along the distribution material in question, such as laterally/dorsally/ventrally. Such distribution materials are sold by, for instance, Muller Textiles, Troy, MI, USA. See EP 2938310 B1 for examples of such distribution materials.

Figures 4a-4e illustrate the use of such distribution layers.

Hence, Figure 4a shows the crotch part 110 comprising, in order from an inner part of the crotch part 110 to an outer part of the crotch part 110, the following layers:
A first distribution layer 122, comprising distribution material of the above-described type having relatively large pores and hence being arranged to transport liquid, via capillary forces, relatively efficiently. In other words, the first distribution layer 122 is arranged to transport, via capillary forces, relatively large volumes of liquid per time unit and per units of distribution material volume;
a second distribution layer 123 of similar type as the first distribution layer 122 but having relatively small pores as compared to the material of the first distribution layer 122;
at least one liquid-absorbing layer 124, that may be as described above; and
a liquid barrier 126, that may be as described above.

Again, it is pointed out that the crotch part 110 may very well comprise additional layers, such as for example more than one absorbing layer 125 in the sense described above in relation to Figures 3a-3c.

Since the first distribution layer 122 distributes liquid more efficiently than the second distribution layer 123, liquid entering into the first distribution layer 122, even locally, will quickly distribute across a larger area of the first distribution layer 122, from where it enters the second distribution layer 123. The second distribution layer 123 may then transport the liquid further down into the liquid-absorbing layer 124 for permanent absorption or absorption until the next washing of the garment 100.

To this end, the first distribution layer 122 may also form an external, entry layer. The first 122 and second 123 layers may be arranged in direct contact to each other, such as bonded together using sparse bonding lines of via a bonding agent that is highly permeable to liquids.

In some embodiments, the first distribution layer 122 may be arranged with higher liquid transportation capacity, as described above, in a direction perpendicular to the skin of the standing user wearing the protective garment 100 as compared to in a direction parallel to said skin, whereas the second distribution layer 123 may be arranged with a higher liquid transportation capacity in a direction parallel to said skin as compared to in a direction perpendicular to said skin.

This provides efficient transportation of the liquid from the skin surface down into the first distribution layer 122 material for further distribution, via the second distribution layer 123, down into the liquid-absorbing layer 124. As a result, the external inside surface of the garment 100 is kept dry while any liquid is quickly and efficiently transported down into the garment and absorbed. In particular, the relatively large-pore first distribution layer 122 may be used in combination with said difference in transportation direction efficiency for an improved effect.

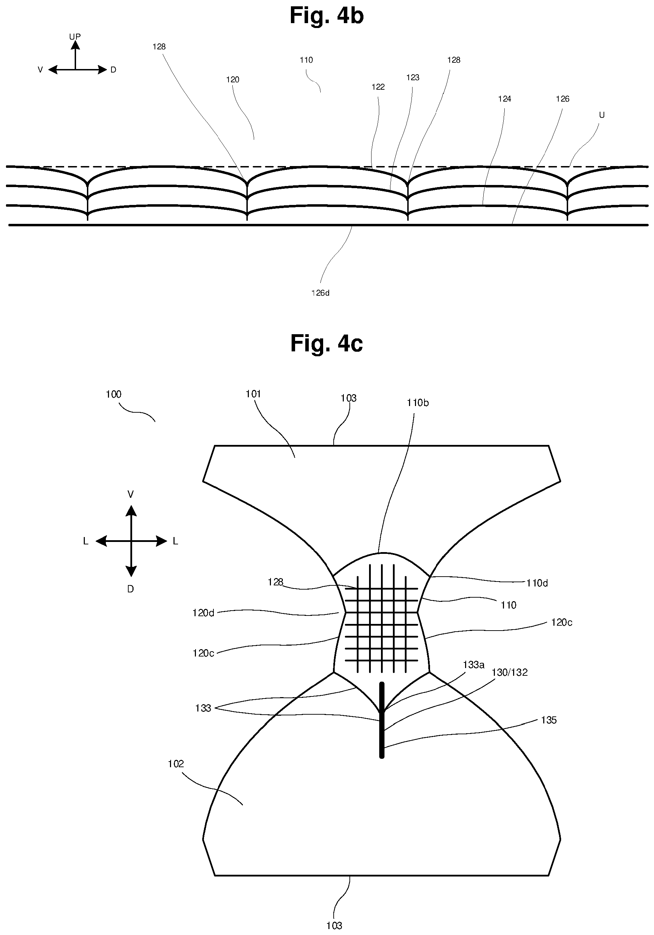

Figures 4b and 4c illustrate an exemplifying embodiment according to which said distribution layers 122, 123 are interconnected using a plurality of connections 128 comprised in the crotch part 110. The connections 128 may be in the form of stitches and/or welds, joining together the above-described first distribution layer 122 to at least one additional layer of the crotch part 110, such as to one or several additional distribution layers and/or one or several liquid-absorbing layers 124, 125.

This plurality of connections 128 then defines a connection pattern, such as a quilted pattern, distributed across the surface of the first distribution layer 122, distributed to cover at least 50% of the first distribution layer 122, at least at a location or area of the genitalia of the standing user wearing the protective garment 100. Hence, the pattern may be distributed to cover at least 50% of the first distribution layer 122 across said central area 112 of the crotch part 110.

The connection pattern may be regular or irregular, and it may be evenly or unevenly distributed across the surface of the first distribution layer 122. In Figure 4c, the pattern is illustrated as an at least locally regular patterns of perpendicular and equidistant bond lines. It is realized that any decorative or non-decorative pattern could be useful, but it is preferred that the pattern is not limited to just a part of, or outside of, an area that is in direct contact with the genitalia of the user wearing the garment 100, such as the central area 112 of the crotch part 110, but that the pattern is distributed to cover at least 50%, or even that entire area.

That the pattern "covers" a certain share of the area in question means that, on a macro level, the area in question is covered by the pattern across such a specified share of its surface, while it is understood that, on a micro level, the pattern itself will contain connections 128 that are separated by sub-areas that are not part of any such connections 128.

Moreover, the connections 128 may preferably constitute a contact-limiting means for limiting a share of the first distribution layer 122 being in direct contact with said user wearing the protective garment 100, by the first distribution layer 122 locally bulging inwards towards the liquid barrier layer 126, away from the skin of the user, at the connection in question. This is illustrated in Figure 4b (the wearing user's U skin being indicated using a broken line), and involves the connections 128 being arranged to locally press together the various layers along bond lines. For instance, the connections 128 may be seams or welds achieving this. This provides for a surface that can efficiently stay dry against the skin of the user while still providing good absorption of liquid, in particular in the case where various combinations of distribution layers 122, 123 are used as described above.

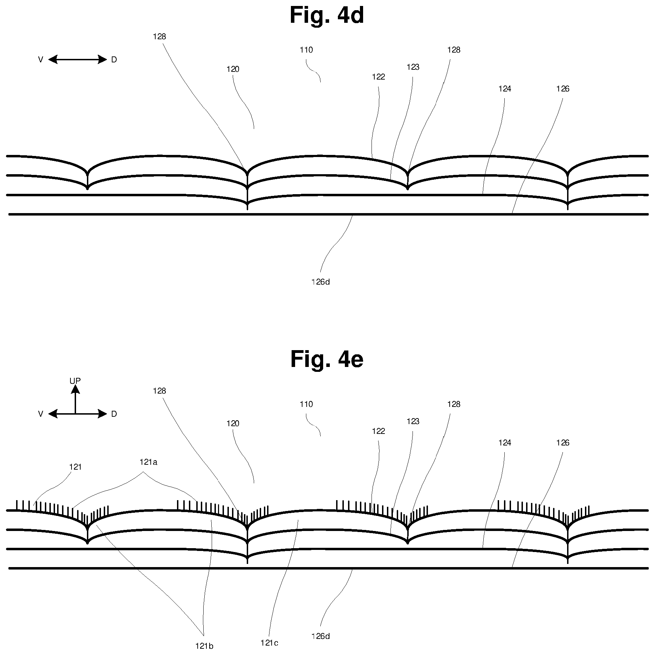

In some embodiments, an example of which is illustrated in Figure 4d, the connection pattern comprises several different connections 128 joining together the first distribution layer 122 with different numbers of other layers, the other layers being selected from the set of the second distribution layer 123; any additional distribution layer comprised in the crotch part 110; the liquid-absorbing layer 124; and any additional liquid-absorbing layers 125 comprised in the crotch part 110. In other words, the connections 128 connect the first distribution layer 122 differently deep into the rest of the material layers.

This way, the surface contact limitation described above can be achieved while still providing sufficient distribution and absorption of liquid entering the first distribution layer 122.

Using such a connection pattern, it is also possible to control various paths along which the liquid is distributed, for instance as a function of an area of the user's body from which the liquid in question originates and/or a total amount of liquid received by the leak-proof part 120.

For instance, the connection pattern may comprise connections 128 surrounding the genitals of the wearing user, forming a wide channel for liquid transportation perpendicular to the user's skin, such connections not penetrating all the way to a particular liquid-absorbing layer, but allowing liquid to be transported, along a deeper distribution layer and beneath said surrounding connections and laterally/dorsally/ventrally out from said wide channel for absorption, allowing the crotch part 110 to absorb varying amounts of liquid and keeping the outer parts of the crotch part 110 completely dry in case of only smaller amounts of liquid. It is noted that this is only one example of a design being possible using such connection pattern of varying connection 128 depth.

It is noted that such differently deep connections 128 may, in various embodiments, also interconnect the inner layer 121 to one or several of the other layers. Moreover, for some or all of the connections 128, the connection 128 in question may bind together two or more layers, none of the bound layers being an interior layer. For instance, in the example shown in Figure 4d, in some locations the first distribution layer 122 may be connected to the second distribution layer 123 without being interconnected to the liquid-absorbing layer 124. In addition or alternatively, in some locations the second distribution layer 123 may be interconnected to the liquid-absorbing layer 124 without being interconnected to the first distribution layer 122.

To further be able to control the distribution of liquid, the connection pattern may comprise hydrophilic and/or hydrophobic connections 128, such as quilted joints comprising stitches using hydrophilic and/or hydrophobic thread. For instance, liquid will only distribute past a hydrophobic connection at a certain minimum liquid pressure, and on the other hand liquid will be drawn slightly to a hydrophilic connection. In the above example with a wide channel, the channel may be sewn with hydrophobic thread forming the corresponding connections, connections within said ring may be sewn using hydrophilic thread. Outside of said ring, a surface arranged to be in direct contact with the skin of a user wearing the garment 100 may be made hydrophobic and/or impermeable to liquids, so that liquid entering through said wide channel and subsequently is distributed laterally/dorsally/ventrally out (in parallel to the skin of said user) from said ring will not be able to resurface into contact with the user's skin outside of said ring.

As mentioned above, the connections 128 preferably do not penetrate through the liquid barrier layer 126, to guarantee liquid-proofness.

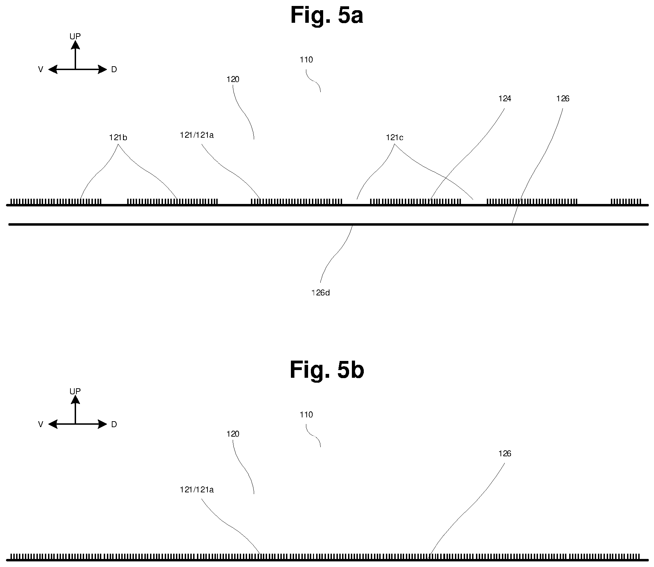

Figure 4e, and also figure 5a, illustrates an embodiment in which the inner surface layer 121 comprises flocked fibres, such as standing or randomly attached fibres 121a on the surface 121. In the Figures, the fibres 121a are illustrated as over-sized standing fibres, for reasons of clarity.

In particular, the flocked fibres 121a may be permanently fastened to the inner surface layer 121 or form the inner surface layer 121. In the latter case, the fibres 121a may be permanently fastened to the distribution layer 122. Alternatively, the flocked fibres 121a may be permanently fastened to the liquid-absorbing layer 124. In other embodiments, the flocked fibres 121a may be permanently fastened to the liquid barrier layer 126.

At any rate, the inner surface layer 121 is permeable to liquid, and the flocked fibres 121a in question are arranged to be in direct contact with the skin of the wearing user. In other words, the flocked fibres 121a may form an external layer.

The permanent fastening in question may be effected by flocking the fibres 121a to a surface of the layer in question, using per se conventional fibre flocking techniques.

Figure 5a shows an example in which the flocked fibres 121a form the inner layer 121 directly on the liquid-absorbing layer 124, and no distribution layers 122, 123 are used.

Figure 5b shows yet another example, in which the flocked fibres 121a form the inner layer 121 directly on the liquid-barrier layer 126, without any distribution 122, 123 or liquid-absorbing layers 124 being used.

It is noted that the flocked fibres 121a may form any pattern, such as any decorative or non-decorative, regular or irregular pattern, uniform or varying. The flocked fibres 121a may also cover the entire surface, or at least substantially the entire surface, such as at least 95% of the surface, of the layer on which they are flocked. The later is preferred in case the fibres are flocked directly onto the liquid barrier layer 126, as is illustrated in Figure 5b.

However, in many embodiments the inner surface layer 121 in question may comprise a flock pattern across the inner surface layer 121, said flock pattern comprising both flocked 121b and non-flocked 121c areas.

Said flock pattern can also be manufactured as, or comprise, local variations in fibre density.

Moreover, the flock pattern can constitute part of the dorsal 130 and/or ventral 130 shaping element(s), for instance by such flocked fibres, due to a combination of flocking density, fibre material properties and/or binding agent material properties, locally increase elasticity or similar.

Preferably, the flocked areas 121b are distributed to constitute at least 20%, such as at least 30%, such as at least 50%, of the surface of the layer to which the flocked fibres 121a are permanently fastened at a location of the genitalia of the standing user wearing the protective garment 100, such as across the above-mentioned central area 112. It is understood that, similarly to the case of the relative coverage of the connection pattern discussed above, the relative coverage of the flocked areas 121b is measured on a macro scale, realizing that the flocked surface may be exposed on the micro scale between individual fibres 121a.

The flocked fibres may all be or comprise hydrophilic fibres, in other words fibres manufactured from a hydrophilic material, such as a hydrophilic plastic material. Particularly in the case of a surface coverage of at least 50%, this is advantageous for liquid absorption in some embodiments, since such hydrophilic fibres attract liquid, guiding it down into the layers underneath the fibres 121a.

Correspondingly, the non-flocked areas 121c may be distributed to constitute at least 10%, such as at least 20%, such as at least 50%, of the surface of the layer to which the flocked fibres 121a are permanently fastened at a location of the genitalia of the standing user wearing the protective garment 100, such as across said central area 112.

In a particularly useful embodiment, hydrophoic fibres are arranged along a peripheral border of the crotch part 110, such as at least along crotch part 110 side edges 110c, along leak-proof body 120 side edges 120c, along absorbing layer 124 side edges 124c and/or along curved fold lines 129a, and preferably also along a respective ventral and a respective dorsal lateral path, so as to define an enclosed area inside said hydrophoic fibres. In addition or correspondingly, one or several connections 128 of the above-discussed type can form liquid-barring lines, arranged to prevent liquid to pass through or across the connection 128 in question in a direction parallel to the skin of the user wearing the garment 100. Such connections 128 may then also be arranged to extend along a peripheral border of the crotch part 110, such as at least along crotch part 110 side edges 110c, along leak-proof body 120 side edges 120c, along absorbing layer 124 side edges 124c and/or along curved fold lines 129a, and preferably also along a respective ventral and a respective dorsal lateral path. Such lines of hydrophoic fibres and/or connections 128 may then form an at least partly closed, preferably completely closed, shape configured not to allow liquid to pass from inside the shape in question to the outside of the shape in question in a direction parallel to the skin of said user. In combination therewith, said inside of said shape may be arranged with partial or complete flocking of hydrophilic fibres on a surface of the crotch part 110 being arranged to be in direct contact with the skin of said user wearing the garment 100. These embodiments are particularly useful in combination with a leak-proof body 120 comprising a spacer material.

Generally, the flocked fibres may all be or comprise hydrophobic fibres. This brings a particular advantage in some embodiments, in particular in case the non-flocked areas 121c have such a minimum relative coverage, since such hydrophobic fibres prevent already contained (inside the leak-proof body 120) liquid to return back up to the surface layer 121.

In particular, a flocking pattern having flocked areas of hydrophilic fibres in combination with other flocked areas of hydrophobic fibres can achieve both rapid entry of liquid into the leak-proof body 120, via the hydrophilic fibres, and limited re-entry of such liquid on the inner external surface of the leak-proof body 120 due to the hydrophobic fibres. In particular, this is the case when used in combination with one or several distribution layers 122, 123 of the above-described type. A pattern comprising both hydrophobic and hydrophilic fibres 121a preferably forms a regular or random pattern covering said central area 112. For instance, the above-discussed example with a wide channel can be arranged this way, with centrally located hydrophilic flocked fibres, perhaps in combination with peripherally located hydrophobic fibres (forming the ring in question).

It is noted that fibres may be flocked directly on a surface of a fabric, as is illustrated in Figures 5a and 5b, or fibres can be flocked onto individual threads or fibres making up said fabric. The bonding agent or adhesive used to fasten the flocked fibres can be hydrophilic or hydrophobic in itself, and be applied across a whole surface to be flocked, or only patch-wise or in a particular pattern. Flocking on individual threads/fibres only requires adhesive to be applied on the individual threads/fibres, allowing holes through the fabric to be formed. Hence, by varying the properties of the flocked fibres and the adhesive, and by flocking on a fabric surface or on individual fibres/threads, a flocked surface that is hydrophobic and liquid-impermeable; hydrophilic and liquid-impermeable; hydrophobic and liquid-permeable; or hydrophilic and liquid-permeable can be created.

Figures 4e-5b illustrate the case in which said flocked fibres comprise external fibres 121a, arranged to be in direct contact with the skin of the standing user wearing the protective garment 100. In this case, the flocked fibres 121a may constitute a contact-limiting means for limiting a share of a layer of the crotch part 110 being in direct contact with said user wearing the protective garment 100. Hence, this way the flocking pattern may work in a way similar to the connection pattern discussed above. As illustrated in Figure 4e, the two can also be used together, forming both a larger-scale contact-limiting means (the connection pattern) and also a smaller-scale contact-limiting means (the flocking pattern), the sum of which provides very high comfort and surface dryness.

Figures 6a-6c illustrate another embodiment, in which the leak-proof body 120 comprises a respective side part 129 arranged on either lateral side of the genitals of a standing user wearing the protective garment 100.

Each such side part 129, that may in itself be leak-proof, such as be made from liquid-impermeable (liquid barrier) material such as a plastic foil, is connected to the rest of the leak-proof body 120 via a respective curved fold line 129a. A centre of curvature of this fold line 129a lies laterally outside of the leak-proof body 120. The fold line 129a may, for instance, describe a circular arc or any other gently bending shape, such as a part of an ellipse-shape.

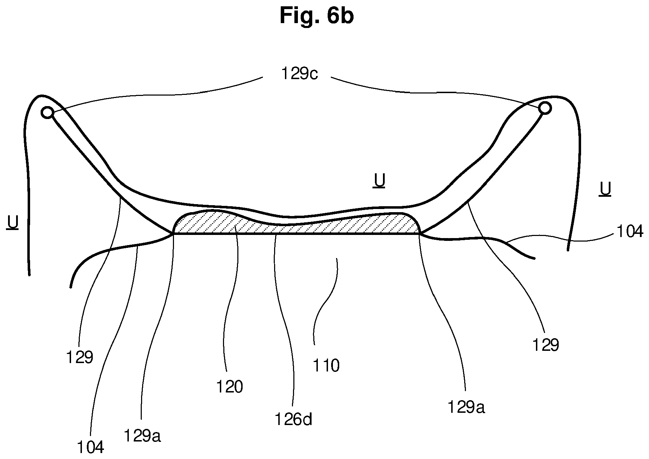

The respective end points 129b of each of said fold lines 129a are interconnected by a flexible element 129d arranged with an elastic property and/or a length resulting in that the flexible element 129d in question applies a force pulling said end points 129b together when the protective garment 100 is worn by the standing user, and as a result of said force pushing the side part 129 in question upwards. As illustrated in Figures 6a and 6b, the flexible element 129d is straight when the garment 100 is worn by the user and the flexible element 129d as a result thereof is stretched or at least subjected to a pulling/tensile force.

Preferably, each of said side parts 129 is only connected to the leak-proof body 120, or even to the rest of the protective garment 100, along said respective curved fold line 129a.

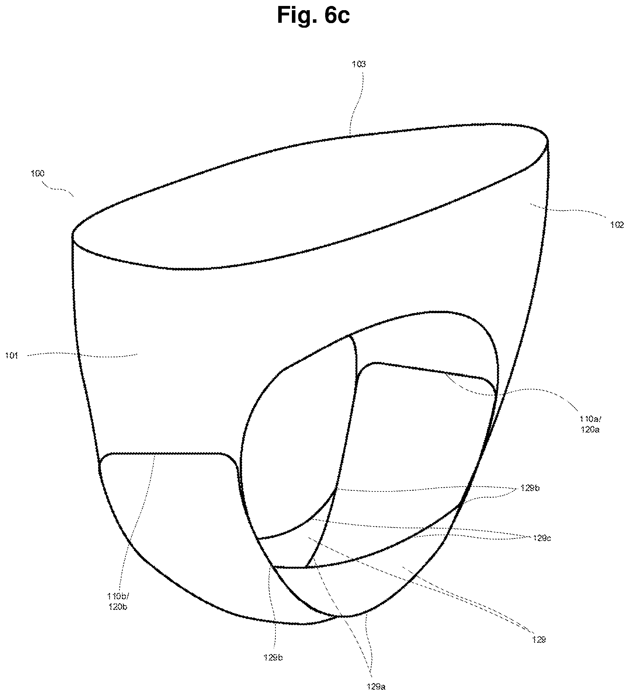

The said pulling force will force said flexible elements 129d upwards in relation to the standing user wearing the protective garment 100, forming a cup-shape covering the genitalia of said standing user wearing the protective garment 100. See Figure 6c.

This provides a very efficient leak-proofing around the genitalia, in particular for a female user, and in particular in combination with the dorsal shaping element 130 and/or the ventral shaping elements 140 as described herein. The present inventor has also come to the conclusion that this provides a particularly good combination between leakage safety and fit for an undergarment 100 of boxer type, having legs extending somewhat down each leg of the wearing user, and in particular in case such legs of such a boxer undergarment 100 are designed to fit loosely rather than stretch around the legs of the user.

It is noted that the side parts 129 do not have to comprise an absorbing layer, but can be provided as a thinner, liquid-impermeable structure.

The leak-proof part 120 may be fastened to the rest of the garment 100, such as to a leg part thereof, along the curved fold line 129a, so that the side part 129 forms a parallel layer to the other part (such as the leg part) of the garment 100 not being a part of the leak-proof body 120, the side part 129 and such other part being free to move one in relation to the other apart along the curved fold line 129a.

A minimum lateral distance between said two fold lines 129a may be at the most 5 cm, such as at the most 4 cm, so that the mentioned cup-shape cups tightly around the genitalia of the female user wearing the garment, as a result of the flexible element 129c pulling the side part 129 upwards to form an upwards slanting structure on either side of the central area 112 (see Figure 6a).

Correspondingly, a minimum lateral distance between said two curved fold lines 129a may be at least 2 cm, such as at least 2.5 cm.

Each of said fold lines 129a may be constituted by a stitched seam, a weld line or any other means locally compressing the material of the leak-proof body 120 along the fold line 129a in question. This is illustrated in Figure 6b, also illustrating a leg part 104 of the protective garment 100 as an example. Alternatively or in addition thereto, the fold line 129a may also be formed by one or several layers having lateral end edges running along the fold line 129a, such as forming the fold line 129a due to this lateral ending. Another option is that the fold line 129a is made using an elongated cut-out of one or several layers, the cut-out being perpendicularly to the fabric.

Furthermore, the fold line 129a may comprise a flocked area of any one of the above-discussed types, such as constituting hydrophobic fibres limiting the distribution of liquid along an inner external surface of the garment 100.

In some embodiments, each of the side parts 129 can be made of a material that is stiffer, such as a thicker plastic foil, as compared to the liquid barrier layer 126.

In some embodiments, each of the side parts 129 are arranged to spring upwards as the crotch part 110 is forced (as a result of the garment being worn by the user) into a shape that curves upwards both ventrally and dorsally. This can be achieved in part by the configuration of the curved fold line 129a, and in part due to the material of the side parts 129 themselves. For instance, each of the side parts 129 may be made from a material achieving that the side part 129 in question springs back into its original shape, such as a flat shape, when not subjected to any external forces. This can be achieved even with soft, comfortable materials, such as a material matrix comprising one or several layers of spacer material of a certain minimum total thickness (such as at least 2 mm), being provided with a liquid-barrier layer, such as a plastic coating, on its outer side.

The fold line 129a may in itself be manufactured so as to be elastic along its longitudinal (dorsal/ventral) direction. For instance, it can be made using a line of extruded silicone both achieving said fold line in the fabric onto which it is extruded and at the same time said elasticity. In another example, the fold line 129a may be manufactured by a corresponding stitched structure, and/or by locally altering a knitting pattern so as to locally achieve a fold line 129a bend of the textile and also a local constriction of the fabric achieving said elasticity. Such an elastic line along the sides of the labia of a wearing femaly user may, apart from constituting a flod line 129a of said type, also constitute the sides of the "Y"-shape described above. Alternatively the sides of the "Y"-shape may also constitute said fold line 129a.

The fold line 129a may also be provided with a high friction surface. For instance, a printed or extruded silicone line may be applied as mentioned, and may be flocked or non-flocked to expose an elongated (dorsally/ventrally) high-friction surface to the skin of the user wearing the protective garment 100.

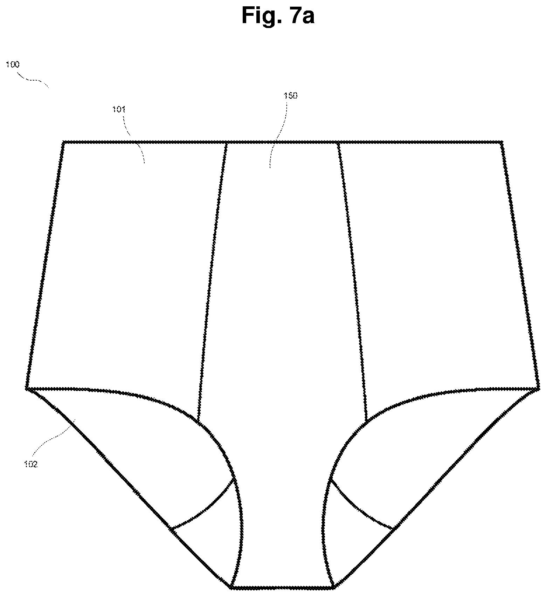

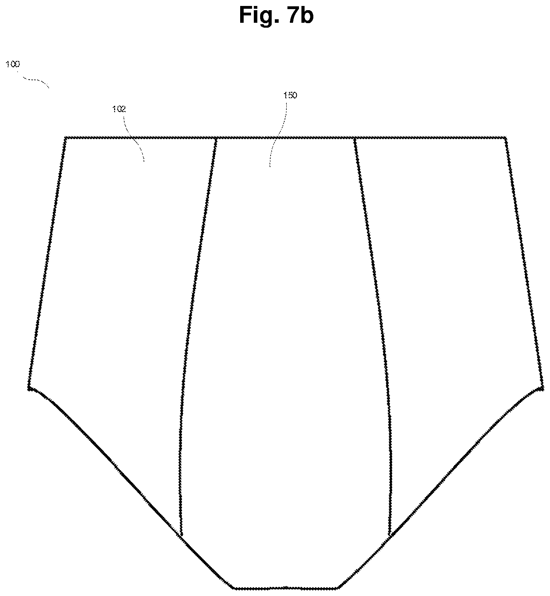

In many embodiments, it is desired to provide a protective garment that can be designed to look the same as a conventional garment, such as a conventional pair of panties. Figures 7a and 7b show a first example of a protective garment 100 designed with this purpose. In this case, the protective garment 100 comprises a cover panel 150, arranged to cover the dorsal shaping element 130 and preferably also any ventral shaping element 140 used. In some embodiments, the cover panel 150 is arranged to cover the crotch part 110 and/or the leak-proof body 120.

In this context, that the cover panel 150 "covers" said parts means that it extends, along a curvilinear plane parallel to the skin of the user wearing the protective garment 100, to cover the part in question completely.

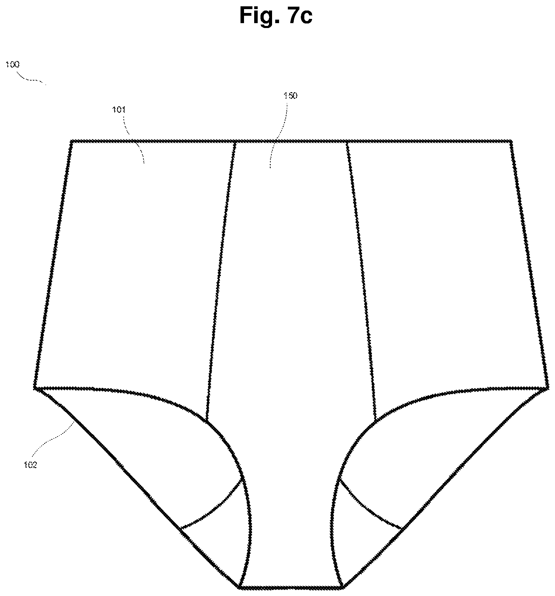

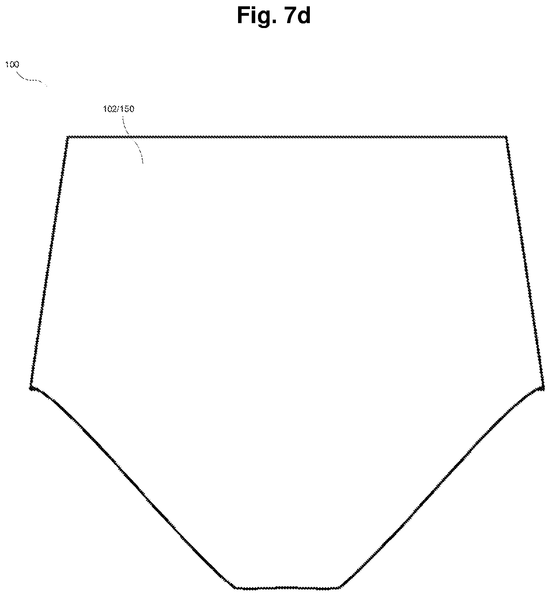

Figures 7c and 7d illustrate a second example of a protective garment 100 comprising such a cover panel 150. Whereas in the example of Figures 7a and 7b the cover panel 150 extends as a generally dorsally-ventrally elongated piece of fabric, from the waist 103 to the waist 103 via the crotch, in the Figures 7c and 7d, the cover panel 150 broadens out dorsally to cover the back part 102 completely. It is understood that these are merely two of many possible examples of how to arrange the cover panel 150.

The cover panel 150 forms an outer external layer in the protective garment 100, visibly hiding the dorsal 130 (and, as the case may be), the ventral 140 shaping element(s). Preferably, the cover panel 150 is sufficiently loose so that a local shape of the shaping element 130, 140 in question is not visible even during use of the protective garment 100 as the wearing user moves around.

The cover panel 150 is preferably fastened to the rest of the protective garment 100 so that it can slide in relation to the shaping element 130, 140 in question. In other words, it is preferably not directly fastened to said shaping element 130, 140.

In its simplest case, the cover panel 150 is just an extra layer of fabric, such as fastened to the rest of the protective garment 150 along its periphery (such as illustrated in Figures 7a-7d), and possibly otherwise not fastened but slidable in relation to the protective garment 100. For instance, the whole front part 101 and/or the whole back part 102 may be constructed with at least two layers of fabric, where an external layer is said cover panel 150 and the shaping element 130, 140 in question is formed on or attached to one or several different layers.

It is understood that such an "extra layer of fabric", as described here, refers to an outer layer irrespectively of how that outer layer fits together with the rest of the garment 100. It may be the case, for instance, that an outer-most layer is integrated with side panels of the garment 100, while an inner layer comprising the dorsal 130 and/or ventral 140 shaping element is fastened to said outer-most layer.

The inner layer may, for instance, be an inner panty-like structure, whereas the "extra layer of fabric" outer layer may be a shorts-like or skirt-like structure, so that the protective garment is a garment (such as a pair of shorts or a skirt) having an outer part generally visible when worn and a built-in protective panty.

The cover panel 150 may be fastened to the rest of the protective garment 100 using stitches, bonding or knitting, in ways generally discussed above.

Above, preferred embodiments have been described. However, it is apparent to the skilled person that many modifications can be made to the disclosed embodiments without departing from the scope of the invention as defined by the appended claims.

It is understood that the various aspects described herein relate to the design and construction of a leak-proof garment which is washable for repeated use, providing superior comfort and that can be designed and constructed to be aesthetically pleasing. Therefore, it is apparent that the various aspects can be combined in many different ways, depending on the detailed requirements and goals, to achieve one or several of these objects.

For instance, using dorsal and/or ventral shaping elements of the present type, and/or side parts of the present type, provides a very tight fit to the wearing user. Using a leak-proof body with various layers of the types described herein, and/or a dorsal and/or ventral joining area of the present type, provides a very good leak-protection and absorption. Any combination between any aspect of the former group of aspects with any aspect of the latter group of aspects will achieve a garment which has a tight fit and also good leak-protection. Since leak-protection is dependent both on a tight fit and an efficient handling of leaking liquids in and in connection to the leak-proof body, there are many synergistic effects by performing such combinations.

Moreover, it is realized that the Figures and examples provided herein are only for illustration of said aspects, and that a protective garment according to the present invention can include many additional parts in addition to the ones described herein, such as ornamental or functional parts.

In addition to the various layers described herein, the garment 100 may furthermore contain one of more layers of a textile net, non-flocked fibres and/or a foam. For instance, an internal layer, directly contacting the skin of the user wearing the garment 100, may comprise an internal textile net or foam material. In other examples, textile layers of different elasticity may be used, for instance to form the dorsal 130 and/or ventral 140 shaping elements as a middle elastic textile layer, defining the shape in question, arranged between two less elastic textile layers. In other examples, the grain direction of different layers of woven fabric may be varied to achieve the desired properties, such as locally achieving a less elastic or even stiff area defining the dorsal shaping element 130 by arranging an enclosed piece of woven fabric there having a grain direction selected to provide relative stiffness in the dorsal-ventral direction.

Hence, the invention is not limited to the described embodiments, but can be varied within the scope of the enclosed claims.

## Claims

1. Protective garment (100) in the form of a pantie, a pair of trousers or other legwear with or without an upper/torso part, the protective garment (100) being a washable garment arranged for repeated use comprising
a front part (101);
a back part (102);
a waist (103); and
a crotch part (110) having a leak-proof body (120),
**characterised in that**
the protective garment (100) comprises a dorsal shaping element (130), arranged along the intergluteal cleft of a standing user wearing the protective garment (100), fastened to or constituting an integrated part of the leak-proof body (120) and fastened to or constituting an integrated part of the back part (102), **in that**
the back part (102) comprises a panel part (102a) separating the dorsal shaping element (130) from the waist (103) so that the dorsal shaping element (130) does not extend all the way up the waist (103) of the protective garment (100) worn by the standing user, **in that** the dorsal shaping element (130) is arranged so that it provides locally differing properties of the material of the protective garment (100) so that, when a dorsal/upward force is applied to the dorsal shaping element (130) via the panel part (102a) as a result of the waist (103) being pulled upwards in relation to said user wearing the protective garment (100), it in turn forces a part of the crotch part (110) extending along the intergluteal cleft of said user upwards, **in that**
said locally differing properties are provided by the dorsal shaping element (130) comprising one or several of applied silicone or a different glue; a weld; a stitching; a fibre flocking; a velvet material; injected molten plastic material, locally varied seamless knitting; and a dorsally elongation of the crotch part (110) overlapping with and fastened to the back part (102), and **in that**
said dorsal shaping element (130) constitutes, is comprised in or comprises a joining area (133), joining together the leak-proof body (120) and the back part (102).

2. Protective garment (100) according to claim 1 or 2, wherein
the dorsal shaping element (130) extends dorsally and upwards along the intergluteal cleft of the standing user wearing the protective garment (100).

3. Protective garment (100) according to any one of the preceding claims, wherein
the protective garment (100) further comprises a pair of elongated ventral shaping elements (140), arranged to extend on either lateral side of the labia of said standing user wearing the protective garment (100), ventrally from the dorsal shaping element (130), and arranged so that, when a ventral/upward force is applied to the ventral shaping elements (140) via the front part (101) as a result of the waist (103) being pulled upwards in relation to said user wearing the protective garment (100), it is in turn forced upwards.

4. Protective garment (100) according to claim 3, wherein said dorsal shaping element (130) is directly connected to said ventral shaping elements (140), wherein
said dorsal shaping element (130), together with said ventral shaping elements (140), may form a "Y"-shape.

5. Protective garment (100) according to any one of the preceding claims, wherein
said dorsal shaping element (130) comprises an integrated part (131) of a knitted or weaved textile layer of the protective garment (100), produced by, during manufacturing, locally varying the properties of said textile layer by changing the weaving or knitting procedure.

6. Protective garment (100) according to any one of the preceding claims, wherein
said joining area (133) joins together a liquid-absorbing layer (124) comprised in the leak-proof body (120) and the back part (102).

7. Protective garment (100) according to any one of the preceding claims, wherein
said dorsal shaping element (130) comprises a part (132) being separate from the crotch part (110) and the back part (102), said separate part (132) connecting the crotch part (110) to the back part (102).

8. Protective garment (100) according to any one of the preceding claims, wherein
said crotch part (110), such as a liquid-absorbing layer (124) comprised in the crotch part (110), comprises a ventral end edge (110b;120b;124b), generally laterally elongated and having a convex shape softly bulging ventrally/upwards in relation to said standing user wearing the protective garment (100).

9. Protective garment (100) according to any one of the preceding claims, wherein
said crotch part (110), such as a liquid-absorbing layer (124) comprised in the crotch part (110), comprises a dorsal end edge (110a;120a;124a), generally laterally elongated and comprising a direction change (134), at the intergluteal cleft of said standing user wearing the protective garment (100), the direction change (134) defining an acute angle (134a) pointing upwards/dorsally in relation to the user.

10. Protective garment (100) according to claim 10, wherein
said dorsal shaping element (130) comprises a straight part (135), extending at least 1 cm, such as between 1 cm and 30 cm, dorsally from the apex of said acute angle (134a).

11. Protective garment (100) according to any one of the preceding claims, wherein
the leak-proof body (120) and/or a central part of the leak-proof body (120) being defined between a pair of dorsally-ventrally extending fold lines (129a) in the leak-proof body (120) has a laterally local narrowest part (120d) arranged between the legs, such as at the gracilis, of the standing user wearing the protective garment (100).

12. Protective garment (100) according to any one of the preceding claims, wherein
the crotch part (110) extends along a path (111) along the sagittal plane of a standing user wearing the protective garment (100), at least part of said path (111) extending along the intergluteal cleft of said standing user, and wherein
the crotch part (110) comprises, in order from an inner part of the crotch part (110) to an outer part of the crotch part (110):
a flexible first layer (121);
a flexible second liquid-absorbing material layer (124), extending along said path (111); and
a flexible third liquid barrier layer (126).

13. Protective garment (100) according to claim 12, wherein
the crotch part (110) comprises a dorsal joining area (133) joining together the first layer (121) and the second layer (124), and wherein
the dorsal joining area (133) extends laterally in relation to said standing user wearing the protective garment (100) and fastens the second layer (124) to the first layer (121).

14. Protective garment (100) according to claim 12 or 13, wherein
the crotch part (110) comprises, in order from an inner part of the crotch part (110) to an outer part of the crotch part (110),
a first distribution layer (122) having relatively large pores;
a second distribution layer (123) having relatively small pores;
a liquid-absorbing layer (124); and
a liquid barrier.

15. Protective garment (100) according to any one of claims 12-14, wherein
the crotch part (110) comprises, in order from an inner part of the crotch part (110) to an outer part of the crotch part (110),
a first distribution layer (122) being arranged with higher liquid transportation capacity in a direction perpendicular to the skin of the standing user wearing the protective garment (100) as compared to in a direction parallel to said skin;
a second distribution layer (123) being arranged with a higher liquid transportation capacity in a direction parallel to said skin as compared to in a direction perpendicular to said skin; a liquid-absorbing layer (124); and
a liquid barrier.

## Patentansprüche

1. Schutzkleidungsstück (100) in der Form einer Unterhose, einer Hose oder einer anderen Beinbekleidung mit oder ohne Oberteil/Rumpfteil, wobei das Schutzkleidungsstück (100) ein waschbares Kleidungsstück ist, das für den wiederholten Gebrauch ausgelegt ist, umfassend einen Vorderteil (101);
einen Rückenteil (102);
einen Bund (103); und
einen Schrittteil (110) mit einem auslaufsicheren Körper (120),
**dadurch gekennzeichnet, dass**
das Schutzkleidungsstück (100) ein Dorsalformelement (130) umfasst, das entlang der Gesäßfalte eines stehenden Benutzers, der das Schutzkleidungsstück (100) trägt, angeordnet ist, das am auslaufsicheren Körper (120) befestigt ist oder einen integrierten Teil desselben bildet und am Rückenteil (102) befestigt ist oder einen integrierten Teil desselben bildet, dass
der Rückenteil (102) einen Verkleidungsteil (102a) umfasst, der das Dorsalformelement (130) von dem Bund (103) trennt, sodass sich das Dorsalformelement (130) nicht vollständig bis zum Bund (103) des Schutzkleidungsstücks (100) erstreckt, das von dem stehenden Benutzer getragenen wird, dass
das Dorsalformelement (130) so angeordnet ist, dass es lokal unterschiedliche Eigenschaften zum Material des Schutzkleidungsstücks (100) bereitstellt, so dass, wenn eine Dorsal/Aufwärtskraft über den Verkleidungsteil (102a) auf das Dorsalformelement (130) ausgeübt wird, als Ergebnis davon, dass der Bund (103) in Bezug auf besagten Benutzer, der das Schutzkleidungsstück (100) trägt, nach oben gezogen wird, dieses wiederum einen Teil des Schrittteils (110), der sich entlang der Gesäßfalte besagten Benutzers erstreckt, aufwärts zwingt, dass
besagte lokal unterschiedliche Eigenschaften durch das Dorsalformelement (130) bereitgestellt werden, umfassend eines oder mehrere von aufgetragenem Silikon oder einem anderen Klebemittel; einer Schweißnaht; einer Naht; einer Faserbeflockung; einem Samtmaterial; eingespritztem geschmolzenem Kunststoffmaterial, lokal variiertem nahtlosem Strickgewebe; und einer Dorsalverlängerung des Schrittteils (110), die den Rückenteil (102) überlappt und mit diesem verbunden ist, und dass
besagtes Dorsalformelement (130) einen Verbindungsbereich (133) bildet, darin umfasst ist oder umfasst, der den auslaufsicheren Körper (120) und den Rückenteil (102) miteinander verbindet.

2. Schutzkleidungsstück (100) nach Anspruch 1 oder 2, wobei
sich das Dorsalformelement (130) dorsal und aufwärts entlang der Gesäßfalte besagten stehenden Benutzers erstreckt, der das Schutzkleidungsstück (100) trägt.

3. Schutzkleidungsstück (100) nach einem der vorstehenden Ansprüche, wobei
das Schutzkleidungsstück (100) ferner ein Paar länglicher Ventralformelemente (140) umfasst, die angeordnet sind, um sich auf beiden Seiten der Schamlippen des stehenden Benutzers, der das Schutzkleidungsstück (100) trägt, ventral vom Dorsalformelement (130) zu erstrecken und
so angeordnet ist, dass, wenn eine Ventral-/Aufwärtskraft über den Vorderteil (101) auf die Ventralformelemente (140) ausgeübt wird, als Ergebnis davon, dass der Bund (103) in Bezug auf besagten Benutzer, der das Schutzkleidungsstück (100) trägt, nach oben gezogen wird, dieses wiederum aufwärts gezwungen wird.

4. Schutzkleidungsstück (100) nach Anspruch 3, wobei
besagtes Dorsalformelement (130) direkt mit besagten Ventralformelementen (140) verbunden ist, wobei
besagtes Dorsalformelement (130) zusammen mit besagten Ventralformelementen (140) eine "Y"-Form bilden kann.

5. Schutzkleidungsstück (100) nach einem der vorstehenden Ansprüche, wobei
besagtes Dorsalformelement (130) einen integrierten Teil (131) einer gestrickten oder gewebten Textilschicht des Schutzkleidungsstücks (100) umfasst, der hergestellt wird, indem, während der Herstellung, die Eigenschaften besagter Textilschicht durch Ändern des Web-oder Strickverfahrens lokal variiert werden.

6. Schutzkleidungsstück (100) nach einem der vorstehenden Ansprüche, wobei
besagter Verbindungsbereich (133) eine flüssigkeitsabsorbierende Schicht (124), die in dem auslaufsicheren Körper (120) enthalten ist, und den Rückenteil (102) miteinander verbindet.

7. Schutzkleidungsstück (100) nach einem der vorstehenden Ansprüche, wobei
besagtes Dorsalformelement (130) einen Teil (132) umfasst, der separat von dem Schrittteil (110) und von dem Rückenteil (102) ist, wobei besagter separater Teil (132) den Schrittteil (110) mit dem Rückenteil (102) verbindet.

8. Schutzkleidungsstück (100) nach einem der vorstehenden Ansprüche, wobei
besagter Schrittteil (110), wie zum Beispiel eine in dem Schrittteil (110) enthaltene flüssigkeitsabsorbierende Schicht (124), eine Ventralendkante (110b; 120b; 124b) aufweist, die im Allgemeinen seitlich verlängert ist und eine konvexe Form aufweist, die in Bezug auf besagten stehenden Benutzer, der das Schutzkleidungsstück (100) trägt, nach ventral/oben sanft gewölbt ist.

9. Schutzkleidungsstück (100) nach einem der vorstehenden Ansprüche, wobei
besagter Schrittteil (110), wie zum Beispiel eine im Schrittteil (110) enthaltene flüssigkeitsabsorbierende Schicht (124), eine Dorsalendkante (110a; 120a; 124a) aufweist, die im Allgemeinen seitlich verlängert ist und eine Richtungsänderung (134) an der Gesäßfalte besagten stehenden Benutzers, der das Schutzkleidungsstück (100) trägt, aufweist, wobei
die Richtungsänderung (134) einen spitzen Winkel (134a) definiert, der in Bezug auf den Benutzer aufwärts/dorsal weist.

10. Schutzkleidungsstück (100) nach Anspruch 10, wobei
besagtes Dorsalformelement (130) einen geraden Teil (135) umfasst, der sich mindestens 1 cm, beispielsweise zwischen 1 cm und 30 cm, dorsal vom Scheitelpunkt besagten spitzen Winkels (134a) erstreckt.

11. Schutzkleidungsstück (100) nach einem der vorstehenden Ansprüche, wobei
der auslaufsichere Körper (120) und/oder ein zentraler Teil des auslaufsicheren Körpers (120), der zwischen einem Paar sich dorsal-ventral erstreckender Faltlinien (129a) im auslaufsicheren Körper (120) definiert ist, einen seitlich lokal engsten Teil (120d) aufweist, der zwischen den Beinen, beispielsweise am Gracilis, des stehenden Benutzers, der das Schutzkleidungsstück (100) trägt, angeordnet ist.

12. Schutzkleidungsstück (100) nach einem der vorstehenden Ansprüche, wobei
sich der Schrittteil (110) entlang eines Pfads (111) entlang der Sagittalebene eines stehenden Benutzers erstreckt, der das Schutzkleidungsstück (100) trägt, wobei sich zumindest ein Teil besagten Pfads (111) entlang der Gesäßfalte besagten stehenden Benutzers erstreckt, und
wobei
der Schrittteil (110) in der Reihenfolge von einem inneren Teil des Schrittteils (110) zu einem äußeren Teil des Schrittteils (110) umfasst:
eine flexible erste Schicht (121);
eine flexible zweite flüssigkeitsabsorbierende Materialschicht (124), die sich entlang besagten Pfads (111) erstreckt; und
eine flexible dritte Flüssigkeitsbarriereschicht (126).

13. Schutzkleidungsstück (100) nach Anspruch 12, wobei
der Schrittteil (110) einen dorsalen Verbindungsbereich (133) umfasst, der die erste Schicht (121) und die zweite Schicht (124) miteinander verbindet, und wobei
der dorsale Verbindungsbereich (133) sich seitlich in Bezug auf besagten stehenden Benutzer, der das Schutzkleidungsstück (100) trägt, erstreckt und die zweite Schicht (124) an der ersten Schicht (121) befestigt.

14. Schutzkleidungsstück (100) nach Anspruch 12 oder 13, wobei
der Schrittteil (110) in der Reihenfolge von einem inneren Teil des Schrittteils (110) zu einem äußeren Teil des Schrittteils (110) umfasst:
eine erste Verteilungsschicht (122) mit relativ großen Poren;
eine zweite Verteilungsschicht (123) mit relativ kleinen Poren;
eine flüssigkeitsabsorbierende Schicht (124); und
eine Flüssigkeitsbarriere.

15. Schutzkleidungsstück (100) nach einem der Ansprüche 12 bis 14, wobei
der Schrittteil (110) in der Reihenfolge von einem inneren Teil des Schrittteils (110) zu einem äußeren Teil des Schrittteils (110) umfasst:
eine erste Verteilungsschicht (122), die mit einer höheren Flüssigkeitstransportkapazität in einer Richtung senkrecht zur Haut des stehenden Benutzers, der das Schutzkleidungsstück (100) trägt, im Vergleich zu einer Richtung parallel zu besagter Haut angeordnet ist;
eine zweite Verteilungsschicht (123), die mit einer höheren Flüssigkeitstransportkapazität in einer Richtung parallel zu besagter Haut im Vergleich zu einer Richtung senkrecht zu besagter Haut angeordnet ist;
eine flüssigkeitsabsorbierende Schicht (124); und
eine Flüssigkeitsbarriere.

## Revendications

1. Vêtement de protection (100) se présentant sous la forme d'une culotte, d'une paire de pantalons ou d'un autre collant avec ou sans partie supérieure/de torse, le vêtement de protection (100) étant un vêtement lavable agencé pour l'usage répété, comprenant :
une partie avant (101) ;
une partie arrière (102) ;
une taille (103) ; et
une partie d'entrejambe (110) ayant un corps étanche aux fuites (120),
**caractérisé en ce que** :
le vêtement de protection (100) comprend un élément de formage dorsal (130) agencé le long du sillon inter-fessier d'une utilisatrice debout qui porte le vêtement de protection (100), fixé à ou faisant partie intégrante du corps étanche aux fuites (120) et fixé à ou faisant partie intégrante d'une partie solidaire de la partie arrière (102), **en ce que** :
la partie arrière (102) comprend une partie de panneau (102a) séparant l'élément de formage dorsal (130) de la taille (103) de sorte que l'élément de formage dorsal (130) ne s'étend pas jusqu'à la taille (103) du vêtement de protection (100) porté par l'utilisatrice debout, **en ce que** l'élément de formage dorsal (130) est agencé de sorte qu'il fournit des propriétés localement différentes du matériau du vêtement de protection (100) de sorte que, lorsqu'une force dorsale/ascendante est appliquée sur l'élément de formage dorsal (130) via la partie de panneau (102a) après que la taille (103) a été tirée vers le haut par rapport à ladite utilisatrice qui porte le vêtement de protection (100), elle force, à son tour, une partie d'entrejambe (110) s'étendant le long du sillon inter-fessier de ladite utilisatrice vers le haut, **en ce que** :
lesdites propriétés localement différentes sont fournies par l'élément de formage dorsal (130) comprenant un élément ou plusieurs parmi la silicone appliquée ou une colle différente ; une soudure ; une couture ; un flocage de fibres ; un matériau en velours ; un matériau en plastique moulé injecté, un tricotage sans couture modifié localement ; et un allongement dorsal de la partie d'entrejambe (110) chevauchant sur et fixé à la partie arrière (102), et **en ce que** :
ledit élément de formage dorsal (130) constitue, est composé de ou comprend une zone d'assemblage (133), assemblant le corps étanche aux fuites (120) et la partie arrière (102).

2. Vêtement de protection (100) selon la revendication 1 ou 2, dans lequel :
l'élément de formage dorsal (130) s'étend de manière dorsale et vers le haut le long du sillon inter-fessier de l'utilisatrice debout qui porte le vêtement de protection (100).

3. Vêtement de protection (100) selon l'une quelconque des revendications précédentes, dans lequel le vêtement de protection (100) comprend en outre une paire d'éléments de formage ventraux allongés (140), agencés pour s'étendre de chaque côté latéral des lèvres de ladite utilisatrice debout qui porte le vêtement de protection (100), de manière ventrale à partir de l'élément de formage dorsal (130), et agencé de sorte que, lorsqu'une force ventrale/ascendante est appliquée sur les éléments de formage ventraux (140) via la partie avant (101) après que la taille (103) a été tirée vers le haut par rapport à ladite utilisatrice qui porte le vêtement de protection (100), elle est à son tour forcée vers le haut.

4. Vêtement de protection (100) selon la revendication 3, dans lequel ledit élément de formage dorsal (130) est directement raccordé auxdits éléments de formage ventraux (140), dans lequel :
ledit élément de formage dorsal (130), conjointement avec lesdits éléments de formage ventraux (140), peuvent former une forme de « Y ».

5. Vêtement de protection (100) selon l'une quelconque des revendications précédentes, dans lequel ledit élément de formage dorsal (130) comprend une partie solidaire (131) d'une couche de textile tricoté ou tissé du vêtement de protection (100), produite, pendant la fabrication, en modifiant localement les propriétés de ladite couche de textile en changeant la procédure de tissage ou de tricotage.

6. Vêtement de protection (100) selon l'une quelconque des revendications précédentes, dans lequel ladite zone d'assemblage (133) assemble une couche d'absorption de liquide (124) comprise dans le corps étanche aux fuites (120) et la partie arrière (102).

7. Vêtement de protection (100) selon l'une quelconque des revendications précédentes, dans lequel ledit élément de formage dorsal (130) comprend une partie (132) qui est séparée de la partie d'entrejambe (110) et de la partie arrière (102), ladite partie séparée (132) raccordant la partie d'entrejambe (110) à la partie arrière (102).

8. Vêtement de protection (100) selon l'une quelconque des revendications précédentes, dans lequel ladite partie d'entrejambe (110), telle qu'une couche d'absorption de liquide (124) comprise dans la partie d'entrejambe (110), comprend un bord d'extrémité ventral (110b ; 120b ; 124b), généralement latéralement allongé et ayant une forme convexe qui se gonfle légèrement de manière ventrale/ascendante par rapport à ladite utilisatrice debout qui porte le vêtement de protection (100).

9. Vêtement de protection (100) selon l'une quelconque des revendications précédentes, dans lequel ladite partie d'entrejambe (110), telle qu'une couche d'absorption de liquide (124) comprise dans la partie d'entrejambe (110), comprend un bord d'extrémité dorsal (110a ; 120a ; 124a), généralement latéralement allongé et comprenant un changement de direction (134), au niveau du sillon inter-fessier de ladite utilisatrice debout qui porte le vêtement de protection (100), le changement de direction (134) définissant un angle aigu (134a) orienté vers le haut/de manière dorsale par rapport à l'utilisatrice.

10. Vêtement de protection (100) selon la revendication 10, dans lequel :
ledit élément de formage dorsal (130) comprend une partie droite (135), s'étendant sur au moins 1 cm, comme entre 1 cm et 30 cm, de manière dorsale à partir du sommet dudit angle aigu (134a).

11. Vêtement de protection (100) selon l'une quelconque des revendications précédentes, dans lequel le corps étanche aux fuites (120) et/ou une partie centrale du corps étanche aux fuites (120) qui est défini(e) entre une paire de lignes de pliage s'étendant de manière dorsale-ventrale (129a) dans le corps étanche aux fuites (120) a une partie locale latéralement la plus étroite (120d) agencée entre les jambes, comme au niveau du muscle droit interne, de l'utilisatrice debout qui porte le vêtement de protection (100).

12. Vêtement de protection (100) selon l'une quelconque des revendications précédentes, dans lequel la partie d'entrejambe (110) s'étend le long d'une trajectoire (111) le long du plan sagittal d'une utilisatrice debout qui porte le vêtement de protection (100), au moins une partie de ladite trajectoire (111) s'étendant le long du sillon inter-fessier de ladite utilisatrice debout, et dans lequel :
la partie d'entrejambe (110) comprend, dans l'ordre à partir d'une partie interne de la partie d'entrejambe (110) jusqu'à une partie externe de la partie d'entrejambe (110) :
une première couche flexible (121) ;
une deuxième couche de matériau d'absorption de liquide flexible (124), s'étendant le long de ladite trajectoire (111) ; et
une troisième couche de barrière au liquide flexible (126).

13. Vêtement de protection (100) selon la revendication 12, dans lequel :
la partie d'entrejambe (110) comprend une zone d'assemblage dorsale (133) assemblant la première couche (121) et la deuxième couche (124), et dans lequel :
la zone d'assemblage dorsale (133) s'étend latéralement par rapport à ladite utilisatrice debout qui porte le vêtement de protection (100) et fixe la deuxième couche (124) à la première couche (121).

14. Vêtement de protection (100) selon la revendication 12 ou 13, dans lequel :
la partie d'entrejambe (110) comprend, dans l'ordre à partir d'une partie interne de la partie d'entrejambe (110) jusqu'à une partie externe de la partie d'entrejambe (110),
une première couche de distribution (122) ayant des pores relativement grands ;
une seconde couche de distribution (123) ayant des pores relativement petits ;
une couche d'absorption de liquide (124) ; et
une barrière au liquide.

15. Vêtement de protection (100) selon l'une quelconque des revendications 12 à 14, dans lequel :
la partie d'entrejambe (110) comprend, dans l'ordre à partir d'une partie interne de la partie d'entrejambe (110) jusqu'à une partie externe de la partie d'entrejambe (110),
une première couche de distribution (122) qui est agencée avec une plus grande capacité de transport de liquide dans une direction perpendiculaire à la peau de l'utilisatrice debout qui porte le vêtement de protection (100) que lorsqu'elle est dans une direction parallèle à ladite peau ;
une seconde couche de distribution (123) qui est agencée avec une plus grande capacité de transport de liquide dans une direction parallèle à ladite peau que lorsqu'elle est dans une direction perpendiculaire à ladite peau ;
une couche d'absorption de liquide (124) ; et
une barrière au liquide.
